(19) Europäisches Patentamt
European Patent Office
Office européen des brevets

(11) **EP 2 997 376 B1**

(12) **EUROPEAN PATENT SPECIFICATION**

(45) Date of publication and mention
of the grant of the patent:
**01.07.2020 Bulletin 2020/27**

(51) Int Cl.:
***G01N 33/574*** *(2006.01)* ***G01N 33/68*** *(2006.01)*
***A61K 31/357*** *(2006.01)* ***A61K 31/58*** *(2006.01)*

(21) Application number: **14725091.4**

(22) Date of filing: **13.05.2014**

(86) International application number:
**PCT/EP2014/059797**

(87) International publication number:
**WO 2014/184211 (20.11.2014 Gazette 2014/47)**

(54) **PROGNOSIS AND PREDICTIVE BIOMARKERS AND BIOLOGICAL APPLICATIONS THEREOF**

PROGNOSTISCHE UND/ODER PRÄDIKTIVE BIOMARKER UND BIOLOGISCHE ANWENDUNGEN DAVON

BIOMARQUEURS DE PRONOSTIC ET/OU DE PRÉDICTION ET APPLICATIONS BIOLOGIQUES CORRESPONDANTES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.05.2013 EP 13305603**

(43) Date of publication of application:
**23.03.2016 Bulletin 2016/12**

(73) Proprietor: **Institut Gustave Roussy
94800 Villejuif (FR)**

(72) Inventors:
• **ROBERT, Caroline
F-92260 Fontenay aux Roses (FR)**
• **BOUSSEMART, Lise
F-75016 Paris (FR)**
• **GIRAULT, Isabelle
F-94800 Villejuif (FR)**
• **VAGNER, Stéphan
F-91370 Verrieres le Buisson (FR)**

(74) Representative: **Cabinet Becker et Associés
25, rue Louis le Grand
75002 Paris (FR)**

(56) References cited:
**WO-A2-2008/082730 US-A1- 2009 186 839**

• **P. ZINDY ET AL: "Formation of the eIF4F Translation-Initiation Complex Determines Sensitivity to Anticancer Drugs Targeting the EGFR and HER2 Receptors", CANCER RESEARCH, vol. 71, no. 12, 15 April 2011 (2011-04-15), pages 4068-4073, XP055082092, ISSN: 0008-5472, DOI: 10.1158/0008-5472.CAN-11-0420**

• **VIRGINIA A ESPINA ET AL: "Phosphoproteomic profiling identifies 4EBP1 and phosphorylated eIF4E as prognostic indicators as well as new drug targets in therapy resistant childhood Rhabdomyosarcoma", PROCEEDINGS OF THE 96TH ANNUAL MEETING OF THE AMERICAN ASSOCIATION FOR CANCER RESEARCH; ANAHEIM, CA, USA; APRIL 16 -20, 2005 , vol. 46 April 2005 (2005-04), pages 1025-1025, XP055082143, Retrieved from the Internet: URL:http://aacrmeetingabstracts.org/cgi/content/abstract/2005/1/1025-a [retrieved on 2013-10-02]**

• **YAN JIA ET AL: "Cap-Dependent Translation Initiation Factor eIF4E: An Emerging Anticancer Drug Target", MEDICINAL RESEARCH REVIEWS, vol. 32, no. 4, 1 July 2012 (2012-07-01), pages 786-814, XP055075039, ISSN: 0198-6325, DOI: 10.1002/med.21260**

• **RYAN J. SULLIVAN ET AL: "Resistance to BRAF-targeted therapy in melanoma", EUROPEAN JOURNAL OF CANCER, vol. 49, no. 6, 2 January 2013 (2013-01-02), pages 1297-1304, XP055082343, ISSN: 0959-8049, DOI: 10.1016/j.ejca.2012.11.019**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

- **WILLIAM H. CHAPPELL ET AL: "Ras/Raf/MEK/ERK and PI3K/PTEN/Akt/mTOR Inhibitors: Rationale and Importance to Inhibiting These Pathways in Human Health", ONCOTARGET, vol. 2, no. 3, 4 March 2011 (2011-03-04), pages 135-164, XP055548291, DOI: 10.18632/oncotarget.240**
- **TERESA LEE ET AL: "Eukaryotic initiation factor 4F: a vulnerability of tumor cells", FUTURE MEDICINAL CHEMISTRY, vol. 4, no. 1, 15 December 2011 (2011-12-15), pages 19-31, XP055493763, GB ISSN: 1756-8919, DOI: 10.4155/fmc.11.150**
- **SCHATZ JONATHAN H ET AL: "Targeted cancer therapy What if the driver is just a messenger?", CELL CYCLE, TAYLOR & FRANCIS INC, US, vol. 10, no. 22, 1 November 2011 (2011-11-01), pages 3830-3833, XP009162646, ISSN: 1538-4101, DOI: 10.4161/CC.10.22.18288**

<u>Remarks:</u>
The file contains technical information submitted after the application was filed and not included in this specification

**Description**

The present invention relates to a method of evaluating the sensitivity or resistance of a cancer cell to a therapeutic treatment. It further relates to the treatment of a cancer with an inhibitor of the eiF4F complex used for the sensitisation of said cancer to a therapeutic treatment.

## BACKGROUND ART

Activation of one or both of the two MAP kinase and PI3 kinase pathways are involved in many cancers including melanoma, renal cell cancer, some colorectal breast and lung cancers for example. For instance, mTOR inhibitors are used to treat RCC and BRAF inhibitors for treating melanoma.

In particular, the Ras/Raf/MEK/ERK pathway is critical for cell survival, growth, proliferation and tumorigenesis. Raf kinases exist as three isoforms, A-Raf, B-Raf and C-Raf. Among the three isoforms, studies have shown that B-Raf (encoded by the BRAF gene) functions as the primary MEK activator and is one of the most frequently mutated genes in human cancers. Thus B-Raf kinase represents an excellent target for anticancer therapy. In particular targeting the MAP kinase pathway by specific anti-BRAF agent is one of the strategies that has demonstrated an unprecedented response rate and an overall survival benefit in patients metastatic melanoma (Flaherty 2010; Chapman et al 2011). This latter targeted therapy can be proposed to the patients, whose melanomas harbour a mutation in position V600 of the *BRAF* oncogene, leading to constitutive activation of the MAPK pathway, which is found in 40 to 50% of melanomas.

Targeted therapy with vemurafenib (Roche /Plexxikon) has shown a survival benefit compared to chemotherapy in these patients (Chapman et al 2011). Although vemurafenib clearly induces early objective responses in more than 50% of the patients, its efficacy is usually of short duration, and almost 100% of the patients relapse after 6 to 12 months of therapy. This is why, although the difference in progression-free survival is very significant (6.8 months vs 1.6 months (hazard ratio 0.38 (95% CI: 0.32-0.46) Log-rank p<0.001), the benefit in overall survival is less impressive: 13.6 months versus 9.7 months (hazard ratio 0.70* (95% CI: 0.57-0.87); p<0.001). For about 15% of the patients, although the melanoma harbors a v600 BRAF mutation, anti-BRAF therapy does not prevent tumor growth. This is what we call primary resistance, in opposition to the secondary resistances occurring in the majority of the initially stabilized or responsive patients.

For secondary resistance, several mechanisms have been identified (Fedorenko, 2011). They are usually classified into two categories:

> 1. those that re-activate the MAPK pathway leading to ERK re-phosphorylation: e.g. amplification or isoform variation of BRAF, upstream Ras-dependent activation of the pathway, downstream ERK activation;
> 2. those that are independent of ERK activation, involving an alternate proliferation pathway, following for example PDGFR or IGF1R activation.

Primary resistances have been much less investigated. Recently, it was shown that the presence of HGF in the microenvironment was a factor of primary resistance in BRAF treated patients via MET, stimulation, capable of activating CRAF, thereby activating the MAP-kinase pathway independently of BRAF. (Straussman, 2012; Wilson, 2012).

Other BRAF inhibitors are being developed, like dabrafenib (GSK) which has demonstrated a similar efficacy in the patients with high response rates (50%), a significant increase in progression-free survival, 5.1 months vs 2.7 with chemotherapy, hazard ratio 0.30 (95% CI: 0.18, 0.51); p<0.0001) and frequent and early development of resistances (Hauschild, 2012). The combination of BRAF and MEK inhibitors is presently the regimen that gives the best response rates with a median time to progression significantly longer than each of the two drugs separately (9.7 months versus 5 to 6 months) (Flaherty, 2012). This combination gives very high levels of initial response rate (up to 76%). However, resistances occur and their underlying mechanisms are less well identified.

Downstream from and at the convergence of these two oncogenic critical pathways: MAPK and PI3K, we find the molecular machinery that regulates gene expression by fine-tuning the processes of mRNA-translation.

This regulation of gene expression is central to providing insights into biological processes. More specifically, the initiation phase of mRNA translation has gained increasing importance for its role in the establishment of correct gene expression patterns during development and diseases. mRNA translation in eukaryotic cells is both physically and temporally separated from transcription. This provides cells with extended options to alter their proteome by potentially imposing a changed translational control over transcripts already present in the transcriptome. "Cap-dependent" mRNA translation relies in part on a complex of proteins called eiF4F, that binds to the 7-methyl-guanine cap located at the 5' end mRNAs (Silvera, 2010; Blagden, 2011). eiF4F comprises 3 main proteins: the cap-binding protein eiF4E, eiF4G that acts as a scaffold and eiF4A, which has an RNA helicase activity. One of the limiting factors is eiF4E, which bioavailability can be modulated by the phosphorylation level of eiF4E-binding proteins (4EBPs). Indeed, eiF4E has been found to be overexpressed in a variety of cancers. It is considered as an oncogene since more than 15 years. The eIF4E-mediated effect on cell transformation and tumorigenesis relies on its ability to increase the translation of a subset of mRNAs that have highly structured 5' untranslated regions (5'UTR). These mRNAs have a weaker ability to compete for eIF4E binding, eIF4E being a rate-limiting factor. Indeed, mRNAs with highly structured 5'UTR, such as those encoding proteins involved in cell cycle progression (MYC, CCND1, ODC), angiogenesis (VEGFA, FGF2) and cell growth and surviving functions, are better recruited to ribosomes and thereby better translated in conditions of eIF4E overexpression.

As stated before, eIF4F is located at the convergence of the two signaling pathways that are critical in tumor biology, and in particular in melanoma biology: the MAPK pathway and the PI3K pathway. While 4EBP1, the eIF4E repressor, is phosphorylated by mTOR (downstream of PI3K/Akt), eIF4E and eIF4G are phosphorylated by Mnk kinases (downstream of MEK/ERK). This has led to the analysis of the expression/phosphorylation of eIF4E, eIF4G and 4EBP1 as predictive/prognostic markers in cancer.

A correlation between the sensitivity or resistance of a tumor to an anti-tumoral agent (such as cetuximab) and the quantity of Cap-OFF complex (4EBP1 protein bound to eiF4E protein) in said tumor has been showed (Zindy, 2011).

The inventors herein show that the assessment of the status of the interaction of eIF4E with eIF4G in the active eiF4F translation initiation complex and with its repressor 4EBP1 is a valuable marker for the evaluation of the sensitivity of a cancer cell to a therapeutic treatment.

## SUMMARY

The present invention is based on the discovery by the inventors that the status of the interaction of cap binding protein eIF4E with its partners eIF4G or 4EBP1 in a cancer cell is correlated to the sensitivity of said cell to a treatment of cancer. By definition, "Cap-OFF" designates herein the complex formed by the interaction of eIF4E with its partner 4EBP1 and "Cap-ON" designates the complex formed by the interaction of eIF4E with its partner eIF4G.

Personalized cancer therapy in particular relies on biomarkers capable of predicting the response of an individual tumor to a treatment of cancer. Advantageous tools usable in the "personalized" follow-up, treatment and cure of a subject having a cancer are herein disclosed.

A method of diagnosing, assessing or monitoring the sensitivity or resistance of a subject having a tumor to a treatment of cancer with an antitumoral agent, preferably an inhibitor of MAPK pathway or the PI3K pathway, more preferably an inhibitor of the MAPK pathway, is herein described. Such a method may advantageously be used to avoid or stop a useless treatment. Such a method of the invention preferably comprises a step of determining, in a biological sample of said subject, in particular in a cancer cell, the Cap-ON or Cap-OFF status of the eiF4F complex has explained below, thereby assessing or monitoring whether the subject having a tumor is responsive or resistant to the treatment of cancer with an antitumoral agent, preferably an inhibitor of MAPK or PI3K pathway, more preferably an inhibitor of the MAPK pathway. The invention relates to the use of Cap-ON or Cap-OFF status of the eiF4F complex, in particular the relative amounts of Cap-ON or Cap-OFF (e.g., the ratio) as explained below for diagnosing, assessing or monitoring the sensitivity or resistance of a subject having a tumor to a treatment of cancer with an antitumoral agent, preferably an inhibitor of MAPK pathway or the PI3K pathway, more preferably an inhibitor of the MAPK pathway.

In particular, the present invention further relates to a method of evaluating sensitivity of a tumor to an antitumoral agent, preferably an inhibitor of the MAPK or PI3K pathway, more preferably an inhibitor of the MAPK pathway, comprising assessing the quantity of eiF4E-eiF4G complex (Cap-ON complex) and the quantity of eiF4E-4EBP complex (Cap-OFF complex) in said tumor or in tumor cells derived therefrom and correlating an increase of the Cap-OFF complex in comparison to the Cap-ON complex with sensitivity to the antitumoral agent, preferably an inhibitor of the MAPK or PI3K pathway, more preferably an inhibitor of the MAPK pathway.

The invention also relates to a method of evaluating resistance of a tumor to an antitumoral agent, preferably an inhibitor of the MAP-kinase pathway or PI3K pathway, more preferably an inhibitor of the MAP-kinase pathway, comprising assessing the quantity of eiF4E-eiF4G complex (Cap-ON complex) and the quantity of eiF4E-4EBP complex (Cap-OFF complex) in said tumor or in tumor cells derived therefrom and correlating an increase of the Cap-ON complex in comparison to the Cap-OFF complex with resistance to the inhibitor of the MAP-kinase pathway.

In a particular embodiment, the tumor is a melanoma, a renal cancer, a colorectal cancer, a breast cancer, a thyroid cancer or a lung cancer. In a preferred embodiment, the tumor is a melanoma, a colon cancer or a thyroid cancer, in particular a melanoma, more particularly a metastatic melanoma. A representative tumor such as a melanoma, a colon cancer or a thyroid cancer is a tumor harboring a mutation in BRAF, in particular a mutation at V600 amino acid position, most particularly the V600E mutation.

The antitumoral agent can be an inhibitor of the MAPK pathway, an inhibitor of the PI3K pathway or an inhibitor of a growth factor or of a growth factor signaling such as an inhibitor of VEGF, or a combination thereof. In a particular embodiment, the treatment corresponds to the administration of an inhibitor of the MAPK pathway, in particular an inhibitor of BRAF, MEK, or MNK or a combination thereof. Representative BRAF inhibitors include in particular inhibitors of a V600E mutant BRAF such as Sorafenib, PLX-4720, Vemurafenib, Dabrafenib, or LGX818. Representative MEK inhibitors include PD0325901, Trametinib, selumetinib and GDC0973.

Preferably, the quantity of the Cap-ON and Cap-OFF eiF4F complexes is assessed using a proximity ligation assay (PLA) or a CAP-binding assay.

Preferably, the method further comprises a step of comparing the relative amount of the Cap-OFF and Cap-ON complexes in the tumor to a reference level. More preferably, the reference level is the amount of the Cap-OFF and Cap-ON complexes in the tumor of the subject before or at the initiation of the treatment with the antitumoral agent (also called pre-therapeutic).

If the subject is identified, using a method according to the present invention, as resistant or non-responder to a particular treatment of cancer, the method advantageously further comprises a step of selecting a "compensatory molecule", to be used, alone or in combination with the particular treatment of cancer as the appropriate therapeutic treatment of cancer for the subject.

A method of selecting an appropriate, preferably optimal, therapeutic treatment of cancer for a subject having a tumor, is therefore in addition herein described, as well as compensatory molecules for use in such a treatment of cancer, preferably in combination with a conventional treatment of cancer in a subject identified using a method as herein described, as resistant to the conventional treatment of cancer.

In a particular embodiment, the method of selecting an appropriate treatment of cancer for a subject having a tumor, comprises a step of determining the ratio of Cap-ON and Cap-OFF eiF4F complexes in a tumor sample of said subject. Preferably, the method further comprises a step of comparing the relative amount of the Cap-OFF and Cap-ON complexes in the tumor to a reference level. More preferably, the reference level is the amount of the Cap-OFF and Cap-ON complexes in the tumor of the subject before or at the initiation of the treatment with the antitumoral agent (also called pre-therapeutic). In particular, the method comprises a step of determining the amount of the Cap-OFF and Cap-ON forms of the eiF4F complex in a biological sample of said subject, a high Cap-OFF/Cap-ON ratio in the biological sample being the indication that a treatment with an antitumoral agent will be efficient in the subject, a low Cap-OFF/Cap-ON ratio in the biological sample being the indication that a treatment with an antitumoral agent will not be efficient in the subject. When a treatment is effective (i.e., a responder subject), it will result in an increase of the Cap-OFF/Cap-ON ratio compared to the pre-therapeutic Cap-OFF/Cap-ON ratio (being preferably the reference ratio for a given patient). If the Cap-OFF/Cap-ON ratio in the tumor does not increase during therapy or decreases during therapy, this indicates that the antitumoral treatment will not be efficient in the subject (i.e., a non-responder subject). In particular, a high ratio is a increased ratio by comparison with the pre-therapeutic ratio and a low ratio is a ratio equal or decreased by comparison with the pre-therapeutic ratio.

In another particular embodiment, the method comprises a step of determining the Cap-OFF/Cap-ON ratio in a tumor sample of said subject, a low ratio, in particular when compared to the pre-therapeutic ratio, being the indication that another molecule for the treatment of the cancer should preferably be administered or added to the tested therapy

Herein disclosed is also a method for screening or identifying a compound suitable for improving the treatment of a cancer (also herein identified as "compensatory molecule") in a subject having a tumor, said method comprising determining the ability of a test compound to increase the Cap-OFF/Cap-ON eiF4F complex ratio.

Further herein disclosed is the use of a compensatory molecule increasing the Cap-OFF/Cap-ON eiF4F complex ratio for treating a cancer or to prepare a pharmaceutical composition for treating a cancer in a subject, identified, by a method as herein described, as resistant to a treatment of cancer, typically to a treatment of cancer with an antitumoral agent, preferably an inhibitor of the MAPK pathway or PI3K pathway, more preferably an inhibitor of the MAPK pathway. The present invention then relates to a compound increasing the Cap-OFF/Cap-ON ratio of the eiF4F complex, for use in a method of sensitizing a subject to a treatment of a cancer with an antitumoral agent, preferably an inhibitor of the MAPK pathway and/or the PI3-kinase pathway, preferably to a BRAF and/or MEK inhibitor as described herein. Preferably, the compound or compensatory molecule is hippuristanol, silvestrol or an analog thereof.

Herein described is, in addition, a method of treating cancer comprising the administration to a subject in need thereof, as previously explained, of a compensatory molecule, preferably together with an antitumoral agent, preferably an inhibitor of the MAPK pathway or PI3K pathway, more preferably an inhibitor of the MAPK pathway, the molecules being administrable together or separately in a common or unique protocol.

Accordingly, the present application further relates to a combination product comprising an inhibitor of the eiF4F complex, in particular hippuristanol or silvestrol or an analog thereof, in combination with an inhibitor of the MAPK pathway and/or the PI3-kinase pathway, preferably an inhibitor of BRAF and/or an inhibitor of MEK, for simultaneous, separate or sequential use in the treatment of a cancer. More particularly, it relates to a combination product comprising hippuristanol or silvestrol, or an analog thereof, in combination to any of the inhibitors provided below, in particular in combination to sorafenib, PLX-4720, vemurafenib, dabrafenib, LGX818, PD0325901, selumetinib, GDC0973 or trametinib.

Also herein disclosed is a method of assessing the prognosis of a cancer in a subject, the method comprising a step of determining, in a biological sample of said subject, the Cap-OFF/Cap-ON eiF4F complex ratio, thereby assessing the prognosis of the cancer in the subject.

Also herein described is a kit for assessing or monitoring the sensitivity or resistance of a subject having a tumor to a treatment of cancer, in particular a treatment with an antitumoral agent, preferably an inhibitor of the MAPK pathway or PI3K pathway, more preferably an inhibitor of the MAPK pathway, or the prognosis of a cancer in a subject, wherein the kit comprises (i) means for assessing the quantity of Cap-OFF and Cap-ON eiF4F complexes, such as means for implementing a proximity ligation assay (PLA) or a CAP-binding assay and, optionally, (ii) a leaflet providing the control quantitative ratio value corresponding to the ratio in a control population which may be a sensitive control population or a resistant control population. The present invention also relates to the use of a kit comprising means for assessing the quantity of Cap-OFF and Cap-ON eiF4F complexes, such as means for implementing a proximity ligation assay (PLA) or a CAP-binding assay, for assessing or monitoring the sensitivity or resistance of a subject having a tumor to a treatment of cancer, in particular a treatment with an antitumoral agent, preferably an inhibitor of the MAPK pathway or PI3K pathway, more preferably an inhibitor of the MAPK pathway.

## LEGENDS TO THE FIGURES

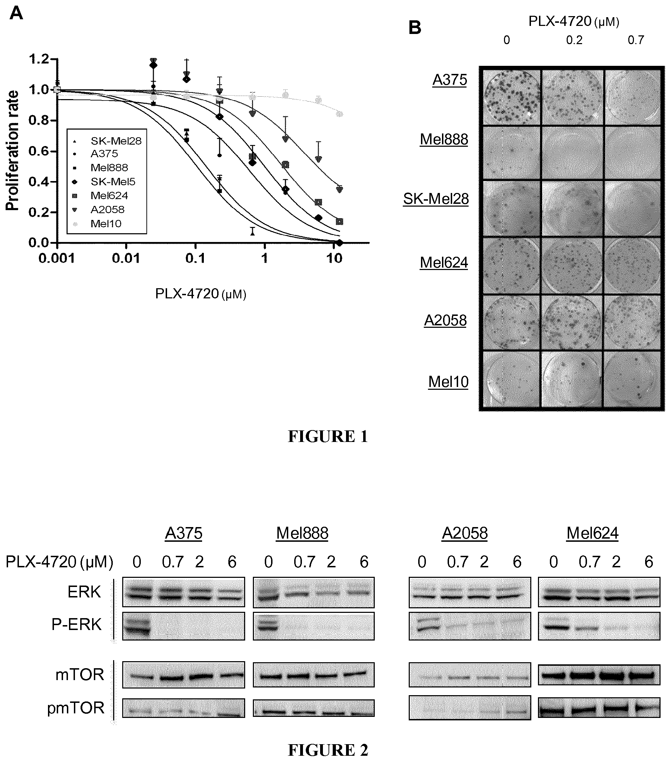

**Figure 1:** Short-term growth-inhibition assay of a panel of melanoma cell lines including BRAF (V600E) cell lines and an NRAS (Q61R) mutated cell line. A. Cells were treated with increasing concentrations of N-[3-[(5-Chloro-1H-pyrrolo[2,3-b]pyridin-3-yl)carbonyl]-2,4-difluorophenyl]-1-propanesulfonamide (PLX-4720) for 72h and cell viability was determined using CellTiter-Blue by measuring the absorbance at 540nm in a microplate reader. Error bars show data $\pm$ standard error. Means were derived from four replicates (n = 4). **B.** Long-term colony formation assay of the indicated cells. Cells were grown in the absence or presence of PLX-4720 at the indicated concentrations for 15-20 days. For each cell line, all dishes were fixed at the same time, stained and photographed.

**Figure 2.** Biochemical responses of the indicated cells to PLX-4720 were documented by western blot analysis for the indicated total and phosphorylated ("P-") proteins.

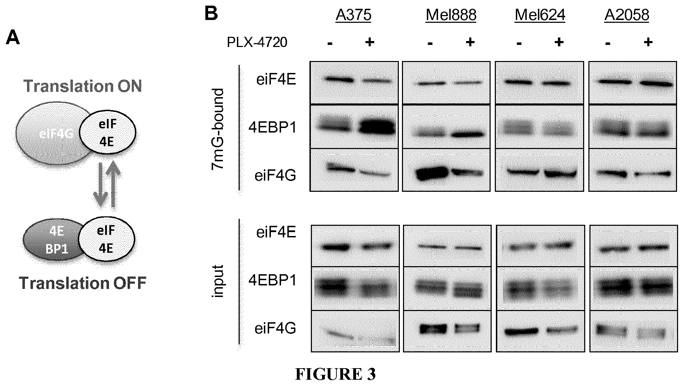

**Figure 3:** Examination of eIF4F complex formation by the « cap binding assay » in the indicated cell lines. Eluates from the beads (m7G-bound) and total extracts (input) were analyzed on 4% to 12% gradient gel electrophoresis, followed by Western blotting with the indicated antibodies.

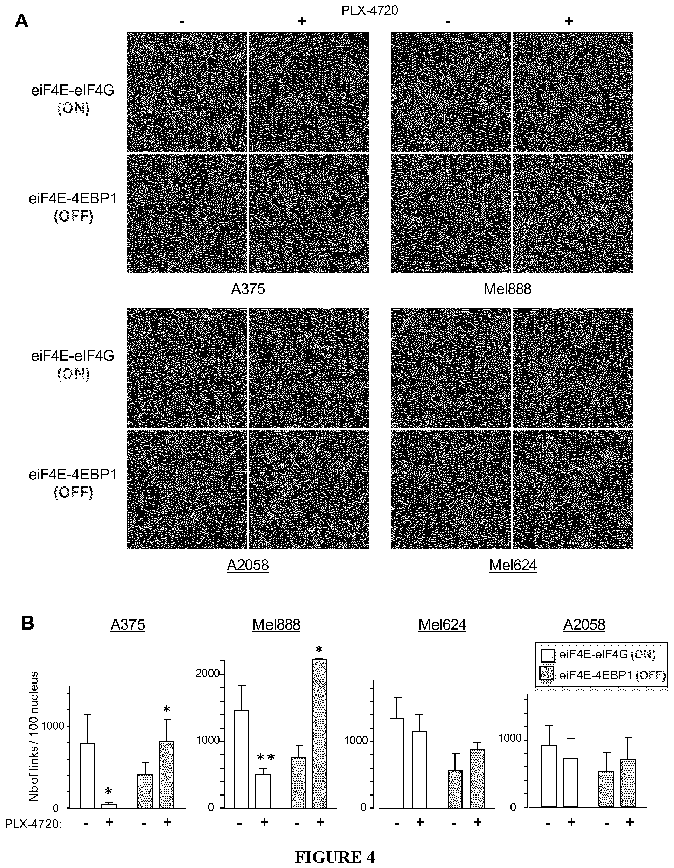

**Figure 4:** The interactions between eIF4E and eIG4G (« Cap-ON ») and between eIF4G and 4EBP1 (« Cap-OFF » interaction) are (A) visualized by the PLA technique and (B) quantified (number of points per 100 nucleus) using ImageJ for the indicated cell lines and treatments.

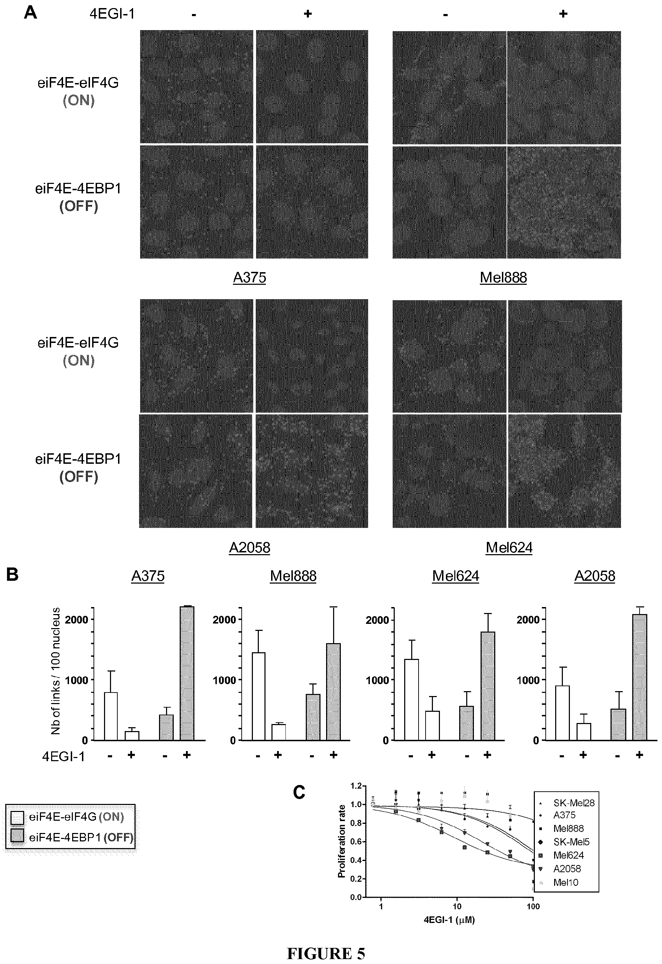

**Figure 5:** The interactions between eIF4E and eIF4G (« Cap-ON ») and between eIF4G and 4EBP1 (« Cap-OFF » interaction) are (A) visualized by the PLA technique and (B) quantified (number of points per 100 nucleus) using ImageJ for the indicated cell lines and treatments. (C) Cells were treated with increasing concentrations of PLX-4720 for 72h, and cell viability was determined using CellTiter-Blue by measuring the absorbance at 540nm in a microplate reader. Error bars show data$\pm$standard error. Means were derived from four replicates (n = 4).

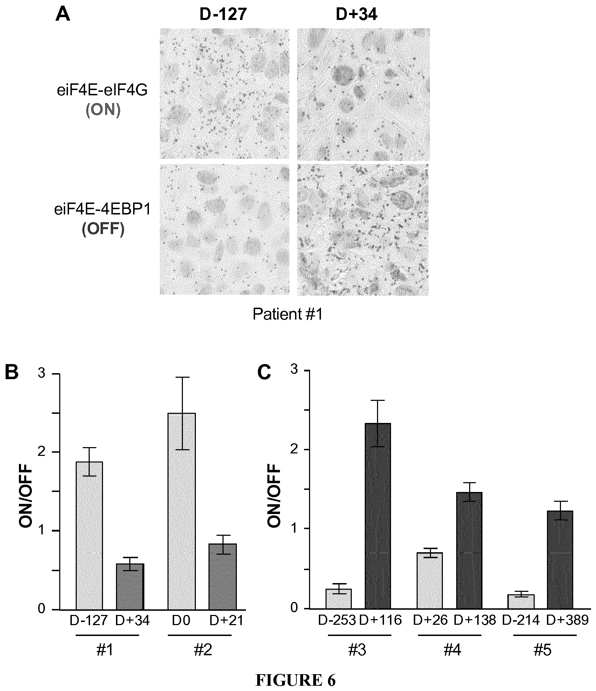

**Figure 6:** The interactions between eIF4E and eIF4G (« Cap-ON ») and between eIF4G and 4EBP1 (« Cap-OFF » interaction) are (A) visualized by the PLA technique in human melanoma formalin-fixed samples from one given patient (named #1). D: Day. The numbers refer to the number of days before (-) or after (+) vemurafenib administration. (B) and (C): Quantification of the "Cap-ON" and "Cap-OFF" interaction (as determined by PLA) on five patients.

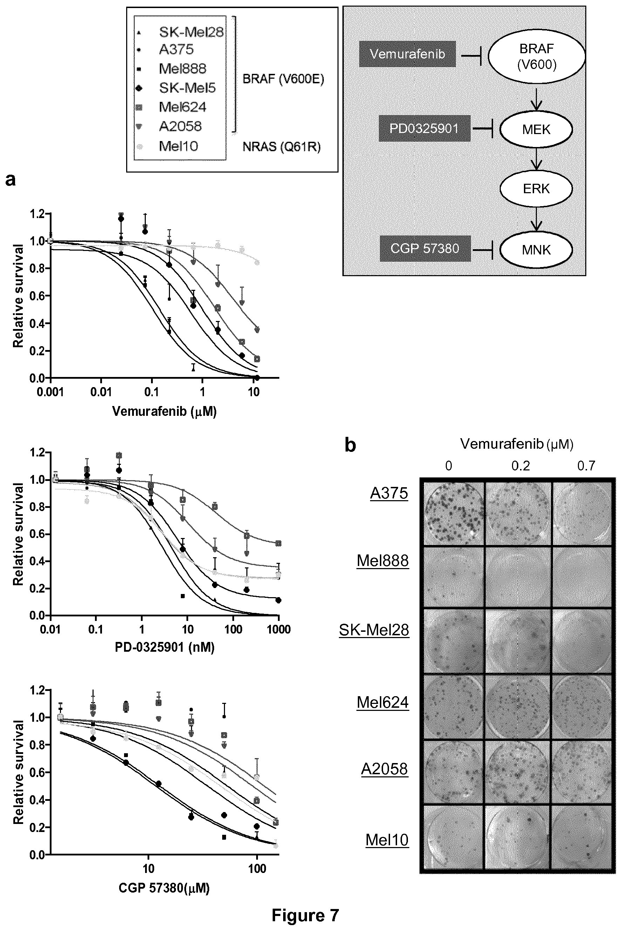

**Figure 7:** a. Short-term growth-inhibition assay of the indicated cell lines (SK-Me128, A375, Mel888, SK-Mel5, A2058, Mel624 and MellO) treated with increasing concentrations of vemurafenib, PD0325901, and CGP 57380 for 48 h. The MellO cell line is not mutated in the BRAF gene and was used as a control vemurafenib-insensitive cell line. Cell viability was determined using the WST-1 cell proliferation assay. Data are means $\pm$ s.d. (n=3). b. Long-term colony formation assay of the indicated cell lines. Cells were grown in the absence or presence of vemurafenib at the indicated concentrations for two weeks. For each cell line, all dishes were fixed at the same time, stained and photographed.

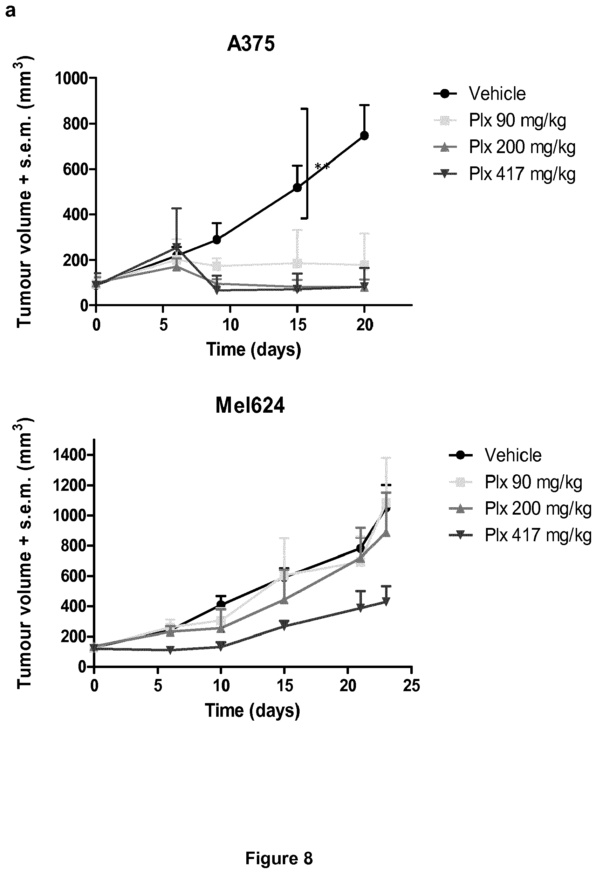

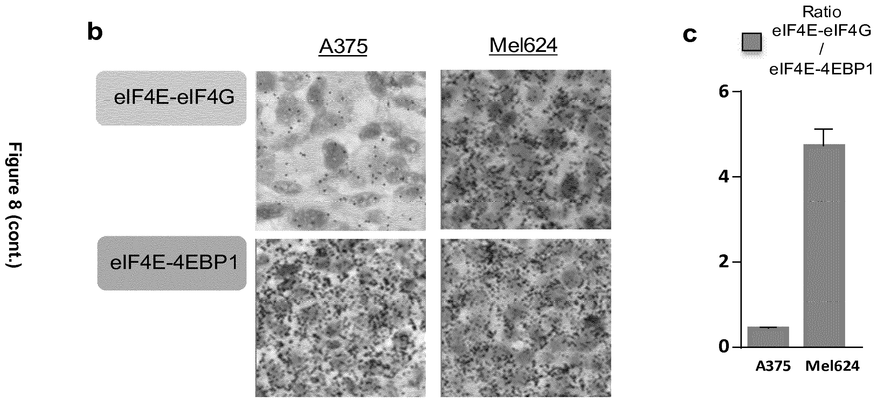

**Figure 8:** a. Growth of A375 and Mel624 cells as tumour xenografts in nude mice treated with vehicle and increasing concentrations of PLX4720 (Plx). Mean tumour volumes +/ s.d.s of the mean are shown (n=3-5 mice per group). Data are means $\pm$ s.e.m. (n=5-6 mice per group). Significance determined by single-sided Mann-Whitney U test (*P<0.05, **P<0.01). b. Interactions between eIF4E and eIF4G (eIF4E-eIF4G) or eIF4E and 4EBP1 (eIF4E-4EBP1) detected by in situ proximity ligation assay in paraffin-embedded tissue sections of A375 and Mel624 xenografts of PLX4720-fed mice. The interactions were visualised as purple fluorescent spots. c. Quantification of the proximity ligation assay showing the ratio between the number of purple spots corresponding to the interactions between eIF4E-eIF4G per 100 nuclei and the number of purple spots corresponding to the interactions between eIF4E-4EBP1 per 100 nuclei, normalized to the same ratio before treatment. Error bars show data $\pm$ s.d.

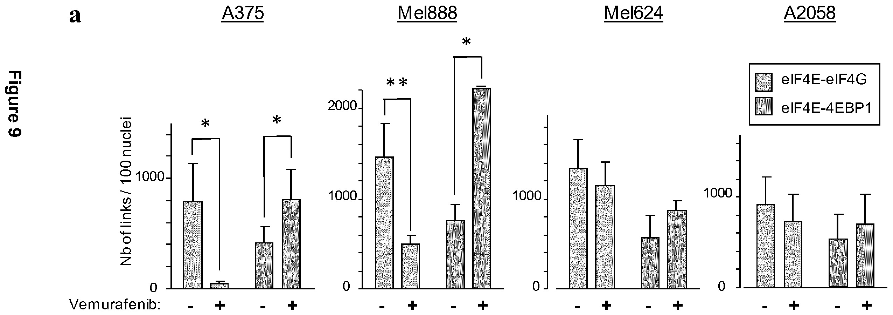

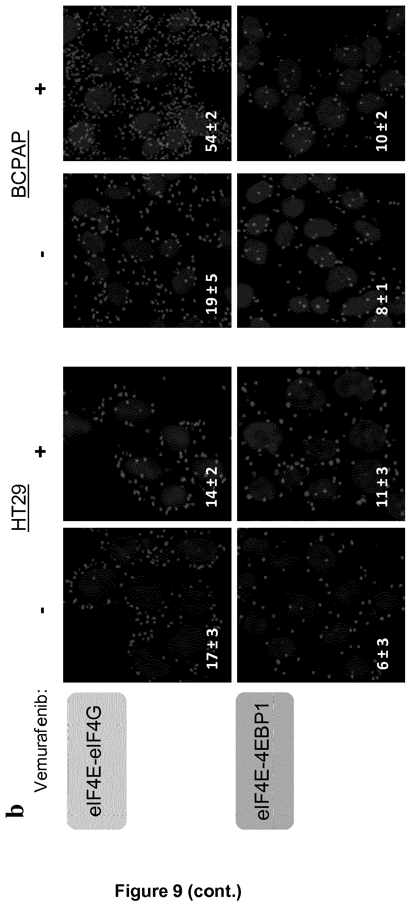

**Figure 9:** The formation of the eIF4F translation initiation complex is associated with resistance to anti-BRAF inhibitors. a. Quantification of the proximity ligation assay showing the number of red spots (corresponding to the interactions between eIF4E-eIF4G or eIF4E-4EBP1) per 100 nuclei (quantified with the Volocity software). Data are means $\pm$ s.d. (n=3). Significance determined by Mann-Whitney U test (*P<0.05, **P<0.01). b. The eIF4E-eIF4G and eIF4E-4EBP1 interactions detected by in situ proximity ligation assay in vemurafenib (6 $\mu$M)-treated (24 h) or untreated HT29 colon cancer cell line and BCPAP thyroid cancer cell line respectively. The interactions were visualised as red fluorescent spots by the scanner Olympus VS120. Cell nuclei were stained with DAPI (blue).

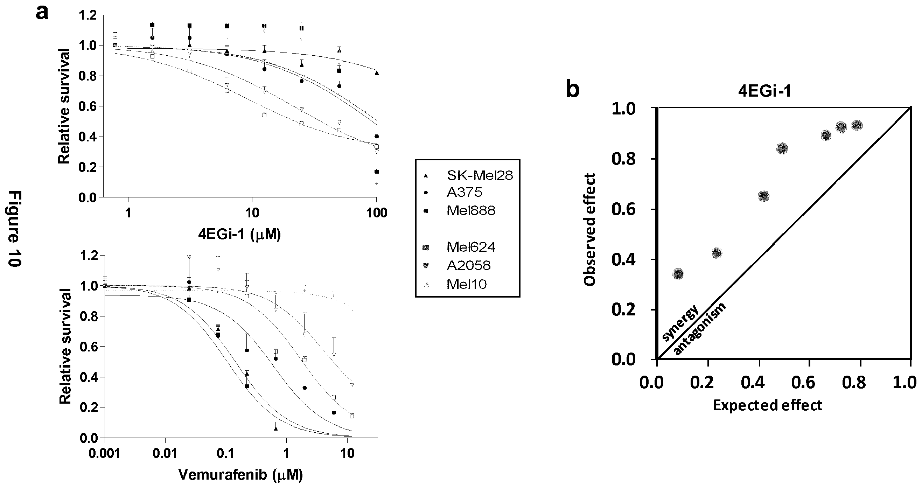

**Figure 10:** Molecules targeting eIF4F synergize with vemurafenib. a. Short-term growth-inhibition assay of the indicated cell lines treated with increasing concentrations of 4EGi-1 (upper panel) and vemurafenib (lower panel) for 48 h. Cell viability was determined using the WST-1 cell proliferation assay. Data are means $\pm$ s.d. (n=4). b. Isobologram showing the correlation between the observed and the expected effects of the combination of vemurafenib and 4EGi-1 in Mel624. The upper left region of the figure represents increasing degrees of synergy. The minimum and maximum Bliss index values were shown as an interval within square brackets under each isobologram. The Bliss index was calculated as the ratio Observed/Expected where an index =1, <0 and >1 indicates additive, antagonistic and synergistic effects, respectively.

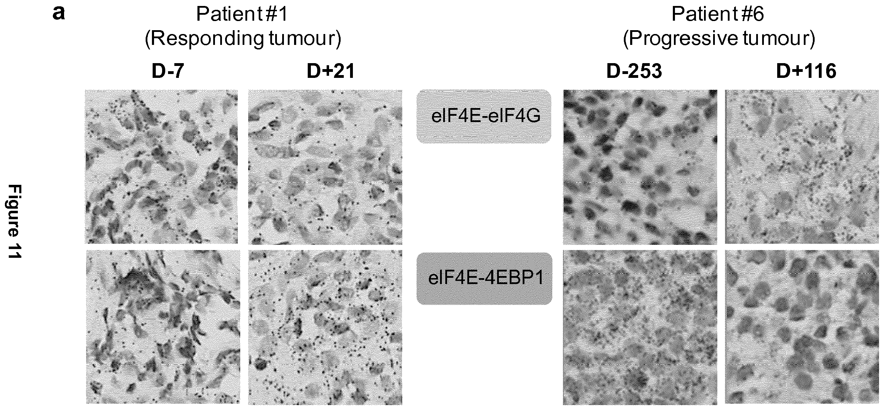

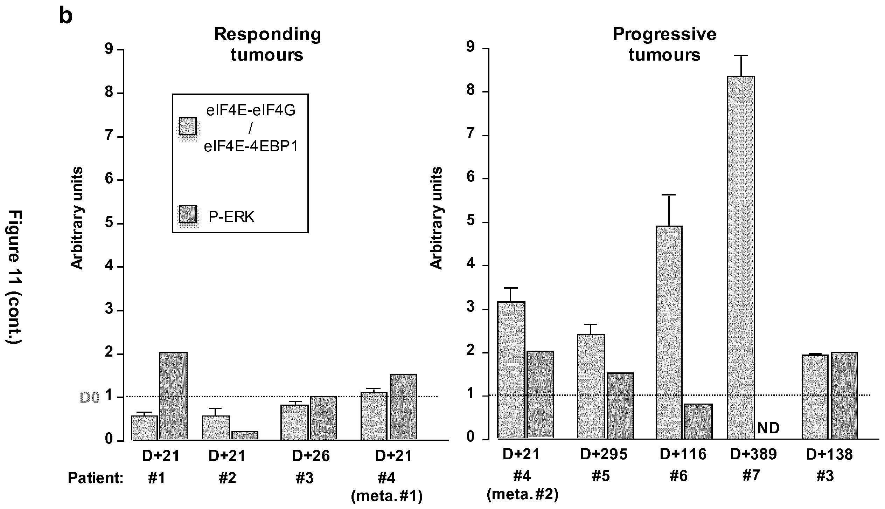

**Figure 11:** Formation of the eIF4F complex in patients. a. The eIF4E-eIF4G and eIF4E-4EBP1 interactions detected by in situ proximity ligation assay in the tumour samples collected from two patients (Patient #1 and #6), before and under vemurafenib treatment. The tumour from patient #1 responded to vemurafenib whereas the tumour from patient #6 was resistant to vemurafenib. Each purple spot represents an interaction. b. Quantification of the proximity ligation assay showing the ratio between the number of purple spots corresponding to the eIF4E-eIF4G interactions per 100 nuclei and the number of purple spots corresponding to the eIF4E-4EBP1 interactions per 100 nuclei, normalized to the same ratio before treatment (Day 0). Error bars show data $\pm$ s.d. For each sample, the phosphorylated ERK immunoreactivity levels were assessed semi-quantitatively (grey bars). Four clinically responding tumours are represented in the left graph, and five clinically resistant tumours are represented in the right graph. Patient #4 had a dissociated response 21 days after initiation of anti-BRAF treatment (M1 was responding while M2 was growing). Patient #3 had a biopsy performed at day 26 during clinical response and at day 138 when relapsing. D: day. The number corresponds to the day of biopsy before (-) or after (+) initiation of anti-BRAF treatment.

## DETAILED DESCRIPTION OF THE INVENTION

The inventors herein below describe means to predictively determine if a subject having a tumor will respond to a treatment of cancer, in particular to a treatment of cancer comprising the administration of an antitumoral agent, preferably an inhibitor of the MAPK or PI3K pathway, or an inhibitor of a growth factor or of a growth factor signaling, more preferably an inhibitor of the MAPK pathway, or will be resistant to said treatment, and/or (ii) to assess the prognosis of a cancer in a subject having a tumor, i.e., to assess whether the subject will survive to the cancer or die from the cancer.

In the context of the present invention, the patient or subject is a mammal having a tumor. In a particular embodiment, the mammal is a human being, whatever its age or sex. However, the veterinary applications are also contemplated by the present invention.

Unless otherwise specified in the present disclosure, the tumor is a malignant or cancerous tumor. In the present invention, the cancer may be any kind of cancer or neoplasia. In a specific embodiment, the tumor is a melanoma, a renal cancer, a colorectal cancer, a breast cancer, a thyroid cancer or a lung cancer. Preferably, the cancer or neoplasia is mediated by the MAPK and/or the PI3K signaling pathways, preferably by the MAPK pathway. In a specific embodiment, the cancer is preferably a melanoma, in particular a metastatic melanoma, more particularly a melanoma harboring a mutation in BRAF, in particular a mutation at V600 amino acid position, most particularly the V600E mutation.

In a particular and preferred embodiment of the present invention, the subject is typically a subject undergoing a treatment of cancer, in particular with an antitumoral agent, preferably one or more inhibitors of the MAPK and/or PI3K signaling pathways, and/or one or more inhibitors of a growth factor or of a growth factor signaling pathway, still preferably one or more inhibitors of the MAPK pathway. This means that, typically, before assessing the prognosis of a cancer in a subject, or before assessing the sensitivity or resistance of the subject to a particular treatment of cancer, this subject has been exposed to said particular treatment of cancer (i.e., said antitumoral agent). The subject may have been exposed to part of a complete conventional treatment protocol, for example to at least one cycle of the all treatment protocol, for example two, three or four cycles of the all treatment protocol.

In another particular embodiment of the present invention, the method of assessing the prognosis of a cancer in a subject or the method of assessing the sensitivity or resistance of a subject to a treatment of cancer is applied on a subject who has not been previously exposed to a conventional treatment of cancer.

The term "biological sample" means any biological sample derived from a patient. Examples of such samples include fluids, tissues, cell samples, organs, biopsies, etc. Most preferred samples are cancer or tumor tissue samples, in particular thyroid, lung, skin, intestine, in particular colorectal, uterus, ovary, breast, renal, lung, bone or head and neck tumor samples. Blood, plasma, serum, saliva, urine, seminal fluid, and bronchoalveolar lavage (BAL), etc, may also be used. Cancer cells from a metastasis or cancer cells obtained from blood as circulating tumor cells may also be used. The biological sample of the subject is preferably selected from a tumor sample. The biological sample may be treated prior to its use, *e.g.* in order to render proteins available. Techniques of cell lysis, concentration or dilution of nucleic acids or proteins, are known by the skilled person.

Within the context of this invention, "non-responder" or "resistant" refers to the phenotype of a subject who does not respond to a treatment of cancer, *i.e.* the volume of the tumor does not substantially decrease, or the symptoms of the cancer in the subject are not alleviated, or the cancer progresses, for example the volume of the tumor increases and/or the tumor generates local or distant metastasis. The terms "non-responder" or "resistant" also refer to the phenotype of a subject who will die from the cancer.

Within the context of this invention, "responder", "responsive" or "sensitive" refers to the phenotype of a patient who responds to a treatment of cancer, *i.e.* the volume of the tumor is decreased, at least one of his symptoms is alleviated, or the development of the cancer is stopped, or slowed down. Typically, a subject who responds to a cancer treatment is a subject who will be completely treated (cured), i.e., a subject who will survive to the cancer. A subject who responds to a cancer treatment is also, in the sense of the present invention, a subject who typically has a much longer disease free survival (DFS) chance than a patient who has been identified, with a method as herein described, as resistant to a treatment of cancer.

The sensitivity or susceptibility of a subject to a treatment of cancer indicates whether the subject is "responder" or "non-responder", in other words whether the subject will or will not, be at least partially treated (tumor growth retardation or regression), preferably be completely treated (cured), by said cancer treatment.

The expression "predicting" herein refers to the likelihood that a subject will respond or not to a treatment of cancer and also the extent of the response. Predictive methods of the invention can be used clinically to make treatment decisions by choosing the most appropriate treatment modalities for any particular subject. Therefore, the present invention also concerns a method for selecting a subject suffering of a cancer for a treatment with a particular antitumoral agent, comprising determining the Cap-OFF/Cap-ON eiF4F complex ratio in a biological sample of said subject, and selecting the subject predicted to be responsive to said treatment.

By definition, Cap-OFF designates the complex formed by the interaction of cap binding protein eIF4E (Eukaryotic translation initiation factor 4E; NCBI Gene ID: 1977; UniProtKB/Swiss-Prot: P06730) with its partner 4EBP1 (eukaryotic translation initiation factor 4E-binding protein 1; NCBI Gene ID: 1978; UniProtKB/Swiss-Prot: Q13541) and Cap-ON designates the complex formed by the interaction of cap binding protein eIF4E with its partner eIF4G (Eukaryotic translation initiation factor 4G ; for example NCBI Gene ID: 1981; for example UniProtKB/Swiss-Prot: Q04637). In the context of the present invention, the terms "eIF4E", "eIF4G" and "4EBP1" include any functional isoform of these proteins.

In a particular aspect, the described method of assessing or monitoring the sensitivity or resistance of a subject having a tumor to an antitumoral agent, preferably an inhibitor of the MAPK pathway and/or PI3K pathway, more preferably an inhibitor of the MAPK pathway, advantageously further comprises a step of comparing the Cap-OFF/Cap-ON ratio in the biological sample of the subject to a reference Cap-OFF/Cap-ON ratio level, for instance the ratio in a reference cell, typically a cancer cell, preferably a cell sensitive to the particular inhibitor(s) used in the treatment. Alternatively, reference or control ratio is determined with a sample of patients or subjects sensitive to the particular treatment. Obviously, the Cap-ON/Cap-OFF ratio can also be considered as an alternative embodiment.

By Cap-OFF/Cap-ON or Cap-ON/Cap-OFF ratio, the important aspect for the present invention is the relative amounts of Cap-OFF and Cap-ON. An high Cap-OFF/Cap-ON eiF4F ratio thus herein in particular refers to an increased amount of the Cap-OFF eiF4F complex relative to the amount of the Cap-ON eiF4F complex in comparison to the relative amount of these complexes in a sensitive cell, and a low Cap-OFF/Cap-ON eiF4F ratio herein refers to a decreased amount of the Cap-OFF eiF4F complex relative to the amount of the Cap-ON eiF4F complex in comparison to the relative amounts of theses complexes in a sensitive cell. However, the person skilled in art understands that other references can be used. For instance, the invention also contemplates a reference corresponding to a ratio determined in a cell resistant to the particular treatment of cancer.

In a preferred embodiment, the reference Cap-OFF/Cap-ON ratio is determined in the same subject before or at the initiation of the treatment with the antitumoral agent. Then, if the Cap-OFF/Cap-ON ratio after or during the treatment with an antitumoral agent increases in comparison with the pre-treatment ratio, then the treatment with the antitumoral agent is effective and the subject is responder. Alternatively, if the Cap-OFF/Cap-ON ratio after or during the treatment with an antitumoral agent doesn't increase or decreases in comparison with the pre-treatment ratio, then the treatment with the antitumoral agent is ineffective and the subject is non-responder.

In the context of the herein described methods, the amount of the different eiF4F complexes may be determined by a number of methods. In particular this amount can be determined by using a method for evaluating protein interaction level, allowing study of the relative amount of active and inactive protein complexes. In particular, this amount may be determined by a proximity ligation assay (PLA) or a CAP-binding assay. Detailed methods are provided in the Example section. A kit is commercially available for PLA (DUOLink kit (OLINK, Uppsala, Sweden).

More generally, the amount of the different eiF4F complexes is determined at the protein interaction level, thereby being clearly distinct from previous disclosures which have focused on RNA and/or protein expression or on phosphorylation status. Indeed, relying on RNA expression can be misleading because of the potential post-transcriptional changes that can occur and induce major discrepancies between the amount of a given RNA, and the amount of the corresponding protein. Studying the level of a given phosphorylated protein can also lead to false conclusions because the activation of the translation machinery is a permanently regulated process, resulting from the balances between the active and inactive complexes. An approach based on the study of the relative amount of active and inactive protein complexes is the one that should give the more precise and immediate indication of the activation/inactivation status of complexes.

An *in vitro* or *ex vivo* method of assessing the prognosis of a cancer in a subject is further herein disclosed. This method comprises a step of determining, in a biological sample of the subject, the Cap-OFF/Cap-ON ratio of the eiF4F complex, thereby assessing the prognosis of the cancer in the subject.

A high Cap-OFF/Cap-ON ratio in a biological sample of a subject, preferably in a tumor sample of the subject, is indicative of a favourable prognosis of the cancer in the subject. On the contrary, the low Cap-OFF/Cap-ON ratio in a biological sample of a subject, is indicative of an unfavourable prognosis of the cancer in the subject.

A low Cap-OFF/Cap-ON eiF4F complex ratio may determine the inability of the subject to positively respond to a cancer treatment to an antitumoral agent such as an inhibitor of the MAPK or PI3K pathway and/or may be used to assess the prognosis of a cancer in a subject.

According to the present invention, a "high Cap-OFF/Cap-ON eiF4F complex ratio" is a ratio higher than 0.6, in particular higher than 0.7, in particular higher than 0.8, in particular higher than 0.9, in particular higher than 1. Conversely, a "low Cap-OFF/Cap-ON eiF4F complex ratio" is a ratio lower than 1, in particular lower than 0.9, in particular lower than 0.8, in particular lower than 0.7, in particular lower than 0.6.

The antitumoral agent may be any agent suitable for treating tumor. It includes the DNA damaging agents, the inhibitors of the MAPK pathway, the inhibitors of the PI3K pathway, the histone deacetylase (HDAC) inhibitors, the tyrosine or serine/threonine kinase inhibitors, the hormonal therapy (e.g., Tamoxifen, Toremifene, Anastrozole, Exemestane, Letrozole, Goserelin/Leuprolide, Megestrol acetate, and Fluoxymesterone), the immunotherapy, inhibitors of a growth factor or of a growth factor signaling such as VEGF or VEGF signaling, or a combination thereof.

DNA-damaging antitumoral agent may in particular be selected from the group consisting of inhibitors of topoisomerases I and/or II, DNA crosslinkers, DNA alkylating agents, and anti-metabolic agents. In a preferred embodiment, the DNA-damaging anti-tumoral agent is chosen from the group consisting of inhibitors of topoisomerases I and/or II, and DNA crosslinkers.

Preferably, the antitumoral agent is an inhibitor of the MAPK (Mitogen Activated Protein Kinase) pathway or the PI3K (phosphoinositide 3-kinase) pathway, or a combination thereof.

In a first preferred embodiment, the antitumoral agent is an inhibitor of the MAPK pathway. Such inhibitors of the MAPK pathway include inhibitors of RAF, in particular BRAF, of MEK1/2 (Mitogen-activated protein kinase, also known as MAP2K) of ERK and of MNK. Preferably, it includes BRAF inhibitors, MEK inhibitors and MNK inhibitors. For instance, such MAPK pathway inhibitors, including MEK and RAF inhibitors, may be selected among the inhibitors known by a person skilled in the art and then may be selected for example among: i) MEK inhibitors: selumetinib, trametinib, AZD6244, R04987655, R05126766, TAK-733, MSC1936369B (AS703026), BAY86-9766, GDC-0973, GDC-0623, PD325901, ARRY-438162, CM 040, E6201, ARRY300; ii) RAF and/or BRAF selective inhibitors: PLX-4720, Sorafenib (BAY-43-9006), vemurafenib, dabrafenib, LGX818, BMS-908662 (XL-281), RAF265, RG-7256 (RO5212054, PLX3603), R05126766, ARQ-736, E-3810, DCC-2036; iii) MNK inhibitors: CGP 57380, CGP 052088, Cercosporamide (Hou et al., 2012). In a preferred embodiment, the inhibitor of the MAPK pathway is selected in the group consisting of sorafenib, PLX-4720, vemurafenib, dabrafenib, LGX818, PD0325901, selumetinib, GDC0973, trametinib and CGP 57380.

In a second preferred embodiment, the antitumoral agent is an inhibitor of PI3K pathway. By inhibitor of PI3K pathway is intended an inhibitor acting on the PI3kinase/AKT/mTOR pathway. In particular, the PI3K inhibitor can also be a mTOR inhibitor, or an Akt inhibitor. For instance, the PI3K inhibitor can be selected in the group consisting of NVP-BEZ235, DC-0941, BGT226, GSK1059615, CAL-101 (GS1101), PX-866, SF1126, INK1117, IPI-145, GDC-0941, BKM120, AMG319, XL147, XL765, Palomid 529, GSK1059615 or GSK615, ZSTK474, PWT33597, TG100-115, CAL263, PI-103, CHR-4432, Archexin, SAR245408, SAR245409, BMK120, GSK2126458, BAY 80-6946, tandutinib, GSK690693, MK-2206, triciribine, PKI-587, BYL719, GDC-0032, PX-478 and GDC-0980.

In another embodiment, the antitumoral agent is an inhibitor of a growth factor or of a growth factor signaling. For instance, the inhibitor of a growth factor or of a growth factor signaling is selected the in the group consisting of a inhibitor of VEGF such as bevacizumab, or of VEGF signaling such as an inhibitor of the VEGFR (e.g. sunitinib, sorafenib or regorafenib) and of other growth factors or growth factor signaling. For instance, the inhibitor of a growth factor or of a growth factor signaling is an inhibitor of EGF or EGF signaling such as an inhibitor of the EGFR (e.g. erlotinib or cetuximab). Other growth factor signaling inhibitors include inhibitors of kit such as imatinib or sunitinib.

Also herein described is a kit for assessing or monitoring the sensitivity or resistance of a subject having a tumor to a treatment of cancer, in particular a treatment with an inhibitor of the MAPK pathway, or the prognosis of a cancer in a subject, wherein the kit comprises (i) means for assessing the quantity of Cap-OFF and Cap-ON eiF4F complexes, such as means for implementing a proximity ligation assay (PLA) or a CAP-binding assay and, optionally, (ii) a leaflet providing the control quantitative ratio value corresponding to the ratio in a control population which may be a sensitive control population or a resistant control population. The kit can further comprise control reagents and other necessary reagents.

The present invention also relates to the use of a proximity ligation assay (PLA) or a kit of the invention for preparing a kit for (i) predictively determining if a subject having a tumor, will respond to a treatment of cancer with an antitumoral agent, preferably an inhibitor of the MAPK or PI3K pathway, more preferably an inhibitor of the MAPK pathway, or will be resistant to said treatment, and/or for (ii) assessing the prognosis of a cancer in a subject having a tumor.

If the subject is identified, using a method according to the present invention, as resistant to a particular treatment of cancer with an antitumoral agent, preferably an inhibitor of the MAPK or PI3K pathway, more preferably an inhibitor of the MAPK pathway, the method advantageously further comprises a step of selecting a "compensatory molecule", to be used, alone or in combination with said antitumoral agent, as the appropriate therapeutic treatment of cancer for the subject.

A method of selecting an appropriate, preferably optimal, therapeutic treatment of cancer for a subject having a tumor, is therefore in addition herein described, as well as compensatory molecules for use in such a treatment of cancer, in a subject identified, using a method as herein described, as resistant to the conventional treatment of cancer.

The inventors have shown that targeting translation initiation may offer a strategy to overcome resistance to antitumoral agents such as anti-BRAF and anti-MEK agents. Therefore, the present invention also relates to the use of a compound inhibit translation initiation to overcome the resistance of a cancer cell to an antitumoral agent. In particular, the invention relates to the use of a compound that inhibits cap-dependent translation by targeting and inhibiting eIF4A.

When a patient remains with a low Cap-OFF/Cap-ON ratio or decreases the Cap-OFF/ap-ON ratio during therapy, meaning a non-response to the treatment, a combination of an inhibitor of the MAPK pathway and an inhibitor of the PI3K pathway can be proposed. Alternatively, an inhibitor of the eiF4F complex, such as silvestrol and analogs, pateamine A, hippuristanol, ribavirin, or 4E-ASO, can be combined to the antitumoral agent, preferably an inhibitor of the MAPK or PI3K pathway or an inhibitor of a growth factor or of a growth factor signaling, more preferably an inhibitor of the MAPK pathway. In a specific embodiment, the present invention relates to a combination of hippuristanol or silvestrol or an analog thereof with a BRAF inhibitor or with a combination of BRAF inhibitor and MEK1/2 inhibitors. Silvestrol and analogs are well-known in the art and for instance disclosed in WO13016658, WO12066002 and WO10060891.

Further herein disclosed is the use of a compensatory molecule as herein described for improving the treatment of a cancer, preferably for treating the cancer, or to prepare a pharmaceutical composition for improving the treatment of a cancer, preferably for treating the cancer in a subject tested and identified, by a method as herein described, as resistant to a treatment of cancer with an antitumoral agent, in particular an inhibitor of the MAPK or PI3K pathway or an inhibitor of a growth factor or of a growth factor signaling such as VEGF or VEGF signaling, preferably of MAPK pathway, in particular to an inhibitor of BRAF or MEK. Preferably, the pharmaceutical composition further comprises, as a combined preparation, another drug used in a treatment of cancer, for simultaneous, separate or sequential use in the treatment of said cancer.

Herein described is, in addition, a method for preventing or treating a cancer comprising the administration to a subject in need thereof, as previously explained, of a compensatory molecule or compound, preferably together with (in combination with) a distinct therapeutic agent, the molecule and distinct therapeutic agent being simultaneously or separately administered in the context of a unique cancer protocol.

Herein disclosed is also a method for screening or identifying a compound suitable for improving the treatment of a cancer (also herein identified as "compensatory molecule") in a subject having a tumor, in particular as provided above a melanoma, said method comprising determining the ability of a test compound (i) to modify the Cap-OFF/Cap-ON ratio of the eiF4F complex. Thereby, compensatory molecules that improve the treatment of a cancer, or reduce the development of a resistance during such a treatment of a cancer could be identified.

In another particular aspect, the method comprises: 1) providing a cell or cell-line sensitive or resistant to an antitumoral agent, preferably an inhibitor of the MAPK or PI3K pathway, more preferably of the MAPK pathway; 2) contacting said cell or cell-line with a test compound; 3) determining the amount of the Cap-OFF and Cap-ON forms of the eiF4F complex, and, 4) selecting the compound which increase the Cap-OFF/Cap-ON ratio in said cell or cell-line.

The compounds identified with one of the herein described screening methods may be used, in the context of the present invention, as compensatory molecules.

In addition, the compound identified with the screening methods of the invention may be used to sensitize the patient to a treatment of cancer with an antitumoral agent, preferably an inhibitor of the MAPK or PI3K pathway, more preferably of the MAPK pathway.

Accordingly, the invention also relates to a method of sensitizing a subject to a treatment of a cancer with an antitumoral agent, preferably an inhibitor of the MAPK or PI3K pathway, more preferably of the MAPK pathway, comprising administering to said subject a compound increasing the Cap-OFF/Cap-ON ratio of the eiF4F complex.

In a particular embodiment, the present invention relates to the use of a compound increasing the amount of the Cap-OFF eiF4F complex relative to the Cap-ON eiF4F complex, for sensitizing a subject in need thereof to an antitumoral agent, preferably an inhibitor of the MAPK or PI3K pathway, more preferably of the MAPK pathway. In particular, the compound is used for sensitizing the subject to a BRAF and/or MEK inhibitor as described above.

More particularly, the invention also relates to a combination treatment of cancer comprising a compound that increases the Cap-OFF/Cap-ON ratio of the eiF4F complex in a tumor or tumor cell and an antitumoral agent, preferably an inhibitor of the MAPK or PI3K pathway, more preferably of the MAPK pathway, and more particularly an inhibitor of BRAF and/or an inhibitor of MEK, for simultaneous, separate or sequential use in the treatment of said cancer. For example, inhibitors of the eiF4F complex such as hippuristanol or silvestrol and analogs thereof as described above could be used in combination to a BRAF inhibitor as described above.

Other characteristics and advantages of the invention are given in the following experimental section, with reference to the figures, which should be regarded as illustrative and not limiting the scope of the present application.

## **EXAMPLES**

EXAMPLE 1:

### *Materials and methods*

### **Cell proliferation**

Cell proliferation was measured using a WST-1 reagent (Roche Applied Science, IN, USA). Melanoma cells were plated with 5000 cells per well in 96-well tissue culture plates. After 24 hours, the cells were treated with drugs or DMSO at the indicated concentrations, in triplicates. WST-1 reagent was added to the wells and incubated at 37°C for 2 hours before and after the treatment period of 48 hours. The plates were then read at 450nm on a Wallac Victor[3] 1420 Multilabel Counter (Perkin Elmer, ON, Canada). Absorbance readings are expressed in terms of cell proliferation as percent of the control cells growth within the 48 hours of treatment.

### **Clonogenic assays**

Cells were plated at low density ($1 \times 10^3$ cells per well in a 6-well tissue culture plates) in fresh media. After 24 hours, the cells were treated with drugs or DMSO at the indicated concentrations, in duplicates. After 9-14 days cells were stained with 0.5% (w/v) crystal violet in 70% ethanol and the number of colonies counted.

### **Cap Binding assay**

Cells were grown for 48 h in 10% Fetal Bovine Serum (FBS) followed by 24 h without FBS. Cells were subsequently grown for 12 h in the absence or the presence of trastuzumab. Cells were harvested and lysed in ice-cold lysis buffer (KCl 150 mM, Hepes pH 7.5 50 mM, DTT 2 mM, Tween 20 0.2 %, and Complete Protease Inhibitor Cocktail from Roche). Cell lysates were incubated with 7-methyl-GTP-sepharose 4B beads (Amersham Biosciences) for 2 h at 4°C. Eluates from the beads (7mG-bound) and total extracts (Tot.) were analyzed on 4-12% gradient gel electrophoresis followed by western blot. Experiments were repeated at least 4 times.

### **Proximity ligation assays**

Proximity ligation assays (PLAs) were performed on both fixed and permeabilized transfected cell lines in chamber slides and TMA slides containing melanoma tissue. Following dewaxing and rehydrating of tissue sections, antigen retrieval was performed by heating the slides for 30 min at 95 °C in TE buffer, pH 9. From this point, the TMA sections and the cell lines were treated identically and the PLA protocol was followed according to the manufacturers' instructions (Olink ioscience, Uppsala, Sweden), with incubation of the primary antibodies at 4°C overnight. After blocking (OLINK), the antibodies were used at the following concentrations: for eIF4E (mouse, clone A-10, SC-271480, Santa Cruz 1:200); for eIF4G (rabbit, 2498; Cell Signaling 1:200); for 4EBP1 (rabbit, clone 53H11; 9644; Cell Signaling 1:200). Proximity ligation assay minus and plus probes PLA (containing the secondary antibodies conjugated with oligonucleotides) were added and incubated 1 h at 37°C. Afterwards, further oligonucleotides are added, allowed to hybridise to the PLA probes, and ligase joins the two hybridised oligonucleotides to a closed circle. The DNA is then amplified (rolling circle amplification), and detection of the amplicons was carried out using the 'Brigthfield detection kit' for the chromogenic revelation or using the 'far Red detection Kit' for the fluoresecence revelation. The sections were mounted with Olink mounting media. The number of in situ PLA signals per cell was counted by semi-automated image analysis.

### Results

To investigate whether the interaction between eIF4E and its partners eIF4G or 4EBP1 may be linked to resistance of melanoma to anti-BRAF inhibitors, the inventors tested a panel of commercially-available BRAF (V600E)-mutated melanoma cell lines for their response to N-[3-[(5-Chloro-1H-pyrrolo[2,3-b]pyridin-3-yl)carbonyl]-2,4-difluorophenyl]-1-propanesulfonamide (or PLX-4720; commercially available at Selleckchem). They identified two cell lines (Mel624 and A2058) that are less sensitive to PLX-4720 than other BRAF (V600E)-mutated melanoma cell lines in both short-term (Fig. 1A) and long-term proliferation assays (Fig. IB).

The inventors quantified the effect of exposing these cell lines to PLX-4720 on the half-maximum inhibitory concentration ($IC_{50}$) (Table 1).

| Table 1: | |
|---|---|
| Cell line | PLX-4720 ($\mu$M) |
| A375 | 0.4 |
| Mel888 | 0.1 |
| SK-Mel28 | 0.4 |
| **Mel624** | **4.4** |
| **A2058** | **2.4** |
| SK-Mel5 | 1.5 |
| Mel10 | >10 |

To address the molecular mechanisms underlying the differences in the sensitivity to PLX-4720 in these cell lines, the inventors tested lysates of PLX-4720-treated cells with phosphoprotein-specific antibodies that identify the activated states of both the MAPK pathway (ERK) and the PI3K-Akt-mTOR pathway. PLX-4720 treatment inhibited ERK phosphorylation downstream of BRAF in a quite similar manner in all tested cell lines (Fig. 2), showing that the differential sensitivity to PLX-4720 cannot be explained by a reactivation of the MAPK pathway. Furthermore, the inventors didn't find a differential effect on mTOR phosphorylation, demonstrating that the difference in sensitivity was not due to an activation of the parallel PI3K-Akt-mTOR pathway.

These data led the inventors to examine the formation of the eIF4F complex in this panel of cell lines. In a so-called "cap binding assay", synthetic m7GTP-Sepharose beads were used to capture eIF4E and its binding partners, eIF4G (called Cap-ON complex) and 4EBP1 (called Cap-OFF complex). The inventors observed that following PLX-4720 treatment, the amount of eIF4E-bound 4EBP1 (Cap-OFF complex) was increased, whereas the quantity of eIF4E-bound eIF4G (Cap-ON complex), *i.e.* the formation of the active eIF4F complex, was decreased in the two PLX-4720-sensitive cell lines (A375, Mel888) (Fig. 3). In contrast, and consistent with the inventors' hypothesis, the formation of the eIF4F complex was unchanged in the two cell lines (Mel624, A2058) with less sensitivity to PLX-4720.

To confirm these data with another assay that might also be more appropriate to evaluate eIF4F complex formation in tumor samples, the inventors took advantage of the "Proximity ligation assay" (PLA) procedure. PLA allows the detection of the cellular distribution of endogenous proteins at a single-molecule level. Paired antibodies are used to detect protein complexes *in situ* in tissues and can be used to assay homo/heterodimerization and protein-protein interactions. As far as the inventors know, PLA has never been used to examine eIF4F formation. They therefore set up this assay to allow detection of both the eIF4E-eIF4G interaction (Cap-ON complex because it is related to the formation of the active eIF4F complex) and the eIF4E-4EBP1 complex (Cap-OFF complex because it is related to the recruitment of eIF4E in a repressed translation complex).

The inventors confirmed the biochemical-based "cap binding assay" procedure with the PLA-based procedure. Indeed, PLX-4720-sensitive cell lines (A375, Mel888) showed reduced signal counts for the eIF4E-eIF4G ("Cap-ON") interaction in the PLX-4720-treated condition as compared to untreated (Fig. 4). In parallel, we observed a PLX-4720-dependent increase in the eIF4E-4BP1 "Cap-OFF" interaction in the same two cell lines. PLX-4720 didn't affect the « Cap-ON » and « Cap-OFF » interaction in the less PLX-4720-sensitive cell lines (Mel624, A2058) (Fig. 4), showing again that, in contrast to PLX-4720-sensitive cell lines, PLX-4720 was not able to alter the formation of the eIF4F in the less sensitive cell lines.

The data presented in Fig. 3 and Fig. 4 led the inventors to conclude that the differential sensitivity to targeted anti-BRAF was associated with the formation of the active eIF4F complex. To demonstrate that the formation of the eIF4F complex is involved in the differential sensitivity of the studied cell lines, the inventors took advantage of the 4EGi-1 compound which was identified following a high-throughput fluorescence polarization binding screen, to inhibit the interaction between eIF4E and eIF4G. 4EGi-1 binds to eIF4E and thereby disrupts eIF4E/eIF4G interaction (Moerke, 2007). The inventors found that 4EGi-1 indeed decreased the eIF4E-eIF4G (« Cap-ON ») interaction and increased in parallel the eIF4E-4BP1 « Cap-OFF » interaction in all tested cell lines, including the two less PLX-4720-sensitive cell lines (Mel624, A2058) (Fig. 5A and B). Furthermore, the inventors found that these two above-mentioned cell lines are more sensitive to 4EGi-1 than the PLX-4720-sensitive cell lines (Fig. 5C), showing that this compound has the potential to circumvent the relative resistance to PLX-4720.

To investigate the formation of the eiF4F complex in patients' tumor samples, the inventors applied the PLA procedure to formalin-fixed samples. They compared the activation status of the status of the "Cap-ON" and "Cap-OFF" interactions in the sequential biopsies before treatment, during therapy and at the development of resistance to vemurafenib. They found that the ratio of "Cap-ON" over "Cap-OFF" complexes decreased during treatment (Fig. 6A and 6B; Patients #1 and #2)) and increased when resistance occurred (Fig. 6C; Patients #3 to #5).

Overall, these results indicate that formation of the cap-dependent eIF4F translation initiation complex is linked to resistance to anti-BRAF agents.

EXAMPLE 2

The inventors have further shown the link of the eIF4F translation initiation complex to resistance antitumoral agents in different cancers with the following experiments.

***Materials and Methods***

**Cell lines and reagents**

The A375, Mel888, A2058, SK-Mel28 and SK-Mel5 melanoma cell lines used in this study were purchased from ATCC and the Mel624 and Mel10 cell lines were a gift from G. Lizee, MD Anderson Cancer Center, Houston and L. Zitvogel, Gustave Roussy, respectively. The BCPAP thyroid cancer cell line and HT29 colorectal cancer cell line were a gift from C. Dupuy and, S. Chouaib, Gustave Roussy, respectively. Cancer cell lines were maintained at 37°C and 5% $CO_2$ in humidified atmosphere and grown in RPMI1640, MEM or DMEM growth media supplemented with 10% of fetal bovine serum, 2 mM glutamine, 50 u/ml penicillin and 50 mg/ml streptomycin (GIBCO). All cell lines were regularly verified to be mycoplasma-free using a PCR based test (Biovalley, France). Cells were treated with a BRAF inhibitor (vemurafenib), MEK inhibitor (PD0325901), MNK inhibitor (CGP 57380), eIF4E-eIF4G interaction inhibitor (4EGi-1). All drugs were dissolved in DMSO for *in vitro* studies. PLX4720 was obtained from Plexxikon without any financial support.

**Proliferation analysis and Bliss index**

Cell proliferation was measured using a WST-1 reagent (Roche Applied Science, IN, USA). Melanoma cells were plated (5000 cells per well) in 96-well tissue culture plates. After 24 h, the cells were treated with drugs or DMSO at the indicated concentrations, in triplicates. WST-1 reagent was added to the wells and incubated at 37°C for 2 h before and after the treatment period of 48 h. The plates were then read at 450 nm on a Wallac Victor[3] 1420 Multilabel Counter (Perkin Elmer, ON, Canada). Cell proliferation is expressed as percent of the absorbance compared to mock-treated cells.

To evaluate the effect of vemurafenib in combination with other drugs, we compared observed and expected responses obtained from the combination treatment. Loewe additivity model was used to predict the combined effect of each drug. The expected effect ($E_{exp}$) of the combination was estimated from each separate drug effect:

$$E_{exp}=E_{drug1}+E_{drug2} -(E_{drug1}.E_{drug2})$$

The ratio observed/expected was calculated according to the Bliss method. In this model the excess above the predicted Bliss index represents the synergistic effect of the combination treatment.

Mel624 cells were treated for 48 h before the WST-1 assay with 4EGi-1 (6 μM) and variable doses of vemurafenib ranging from 25 nM to 12000 nM.

## Clonogenic assays

For clonogenic assays, cells were plated at low density ($1\times10^3$ cells per well in a 6-well tissue culture plates) in fresh media. After 24 h, cells were treated with drugs or DMSO at the indicated concentrations, in duplicates. After 14 days cells were stained with 0.5% (w/v) crystal violet in 70% ethanol and the number of colonies was counted.

## Proximity Ligation Assay

Proximity ligation assays (PLAs) were performed on both fixed/permeabilized cells and melanoma tissue sections. Following dewaxing and rehydration of tissue sections, antigen retrieval was performed by heating the slides for 30 min at 95 °C in TE buffer, pH 9. From this point, the tissues sections and the fixed/permeabilized cells were treated identically and the PLA protocol was followed according to the manufacturers' instructions (Olink Bioscience, Uppsala,Sweden), with incubation of the primary antibodies at 4°C overnight. After blocking, the antibodies were used at the following concentrations: for eIF4E (mouse, clone A-10, SC-271480, Santa Cruz 1:200); for eIF4G (rabbit, 2498; Cell Signaling 1:200); for 4EBP1 (rabbit, clone 53H11; 9644; Cell Signaling 1:200). PLA minus and PLA plus probes (containing the secondary antibodies conjugated with oligonucleotides) were added and incubated for 1 h at 37°C. Afterwards, more oligonucleotides were added and allowed to hybridise to the PLA probes. Ligase joined the two hybridised oligonucleotides to a closed circle. The DNA was then amplified (rolling circle amplification), and detection of the amplicons was carried out using the 'Brigthfield detection kit' for the chromogenic revelation or using the 'far Red detection Kit' for the fluorescence revelation. The sections were mounted with Olink mounting media. The first results were visualised by confocal microscopy (SPE (leica)) and the analysis supported by the Volocity software. To improve the sensibility of the fluorescence detection, we next use a scanner (Olympus VS120) and the number of PLA signals per cell was counted (>3 fields) by semi-automated image analysis (ImageJ and oLyvia).

## Mouse xenograft study

Animals were housed under pathogen-free conditions with food and water *ad libitum.* Experiments were performed in accordance with CCAC guidelines and approved by the ethical committee of the "Plateforme d'évaluation Préclinique" of Gustave Roussy. Female Balb/c athymic nude mice (nu/nu) were purchased from the animal facility of Gustave Roussy. At the age of 6-8 weeks, they were injected subcutaneously in the right flank with $5x10^6$ A375 cells in 200 $\mu$l PBS or $5x10^6$ Mel624 cells in 200 $\mu$l PBS-50% matrigel (BD Biosciences). When tumours reached an average volume of 100-300 mm$^3$, mice were fed with control rodent diet or 90 (low dose), 200 (medium dose) or 417 mg/kg (high dose) of the vemurafenib analog PLX4720 (Plexxikon, USA). Tumour growth was monitored twice a week in two dimensions using a digital caliper. Tumour volumes were calculated with the ellipsoid volume formula L x w$^2$ x 0.5 (L=Length; w=width).

## Immunohistochemistry

Deparaffinised tissue sections were treated with Antigen Retrieval Solution (Citrate buffer pH 6.0, concentrated 10X, T0050 Diapath, EDTA buffer pH 9.0, concentrated 10X T0100 Diapath). Tissue sections were then incubated with Peroxidase Blocking Reagent (Peroxidase blocking solution S2023, Dako) for 15 min and Protein Block (Protein block serum-free ready to use X0909 Dako) for 20 min. Primary antibody against phosphorylated (Thr202/Tyr204) ERK1/2 (rabbit, clone 20G11,4376, Cell Signaling) was applied, and the slides were incubated overnight at 4°C. Signals were visualised using rabbit HRP-conjugated secondary antibody (Dako, K4003) and a haematoxylin (Hematoxylin solution Mayer's MHS32) counterstain.

## *Results*

To further investigate the potential role of eIF4F complex formation in the resistance of BRAF(V600) melanoma to anti-BRAF and anti-MEK compounds, we first tested a panel of melanoma cell lines (Table 2) for their sensitivity to vemurafenib.

Table 2:

| | | | A375 | Mel888 | SKMEL 28 | Mel624 | A2058 | MEL10 |
|---|---|---|---|---|---|---|---|---|
| IC50 | Anti-BRAF V600E | Vemurafenib ($\mu$M) | 0,4 | 0,1 | 0,4 | 2 | 2,4 | >10 |
| | Anti-MEK | PD 0325901 (nM) | 10 | 2 | 9 | 96 | 33 | 3 |
| | Anti-Mnk | CGP 57380 ($\mu$M) | 35 | 25 | 60 | >100 | >100 | 28 |
| | Anti-eIF4F | 4EGi-1 ($\mu$M) | >100 | 77 | >100 | 26 | 50 | >100 |
| | | Silvestrol (nM) | 21 | 17 | 3 | 4 | 2 | 46 |

Two BRAF(V600)-mutated cell lines (Mel624, A2058) were resistant to vemurafenib, compared to other cell lines (*e.g.* A375, Mel888) in both short-term (Fig. 7a) and long-term proliferation assays (Fig. 7b). Similarly to the sensitivity pattern observed *in vitro*, A375 xenografts were more sensitive to vemurafenib than Mel624 xenografts (Fig. 8a). Determination of half-maximum inhibitory concentration ($IC_{50}$) (Fig. 7a, Table 2) and long-term proliferation assays (Fig. 7b) indicated that these two vemurafenib-resistant cell lines (Mel624 and A2058) were also less sensitive to PD0325901, a MEK inhibitor, and to the MNK inhibitor CGP 57380 (Fig. 7a). To determine the status of the eIF4F complex in these various cell lines we used a cap-binding assay where synthetic m7GTP-Sepharose beads capture eIF4E and its two binding partners eIF4G or 4EBP1. We observed that vemurafenib led to an increase in the amount of eIF4E-bound 4EBP1 and to a concomitant decrease in the amount of eIF4E-bound eIF4G in the two sensitive cell lines (A375, Mel888).

Strikingly, the vemurafenib-dependent disruption of the eIF4E-eIF4G complex was not observed in the Mel624 and A2058 resistant cell lines. We further investigated the formation of the eIF4F complex using a proximity ligation assay procedure and we visualised that vemurafenib did indeed induce a decrease in eIF4E-eIF4G interaction and an increase in the formation of eIF4E-4EBP1 complexes in vemurafenib-sensitive cell lines (A375, Mel888) that was not observed in the resistant cell lines (Mel624, A2058) (Fig. 9a). *In vivo* experiments in mice were consistent with these findings as we also found fewer eIF4E-eIF4G interactions, as compared to eIF4E-4EBP1 interactions, in vemurafenib-treated A375 xenografts (sensitive) than in vemurafenib-treated Mel624 xenografts (resistant) (Fig. 8b and c).

Finally, this paradigm was not limited to melanoma cell lines as the maintenance of the eIF4F complex was also observed in the colon cancer cell line HT29, in which resistance is associated with an activating PI3K(P449T) mutation (Oikonomou et al., 2011) and EGFR overexpression (Prahallad et al., 2012) as well as in the papillary thyroid cancer cell line BCPAP, where resistance is linked to HER3 overexpression (Montero-Conde et al., 2013) (Fig. 9b).

Vemurafenib-resistant cell lines were more sensitive to 4EGi-1 than vemurafenib-sensitive cell lines (Fig. 10a), suggesting a direct role of the eIF4F complex in their proliferating properties. To examine whether resistance could be reversed by disrupting the eIF4F complex, we explored the effect of the combination of vemurafenib and 4EGi-1 on Mel624 and found a clear synergistic effect (Fig. 10b). Thus, the formation of the eIF4F complex is directly involved in sensitivity to vemurafenib. Targeting translation initiation may therefore offer a strategy to overcome resistance to anti-BRAF/anti-MEK compounds.

In order to evaluate the clinical relevance of the eIF4F complex in patients, we investigated its presence in sixteen biopsy specimens (before and under anti-BRAF therapy) from seven patients with metastatic melanoma. We observed a decrease in eIF4F complex formation (as measured by the ratio of the eIF4E/eIF4G complex compared to the eIF4E/4EBP1 complex) in three out of four biopsy samples of responding tumours (Fig. 11) as compared to biopsy samples taken prior to therapy (baseline). In contrast, in all resistant tumours progressing under therapy, we found a clear increase in eIF4F complex formation, compared with baseline biopsy samples (Fig. 11). Interestingly, patient #4 had two metastases with an early-dissociated response to vemurafenib treatment, one metastasis responding to treatment (M1) and another resisting treatment (M2). While an increase in eIF4F was found in the resistant #M2 metastasis, no change was observed in the responding #M1 metastasis (Fig. 11b). Thus a direct correlation was found between the response to treatment and the formation of eIF4F, illustrating the *in vivo* association between sensitivity to anti-BRAF therapy and inhibition of the eIF4F translation initiation complex. We found that the increase in eIF4F complex formation is more significantly associated with resistance than the ERK phosphorylation level which is conventionally investigated to explore resistance (Fig. 11b). This confirmed our *in vitro* data which showed that the formation of the eIF4F complex was associated with resistance to vemurafenib in both ERK-dependent and ERK-independent resistance mechanisms. Altogether, our data indicate that the formation of the eIF4F complex might be a more reliable resistance biomarker than ERK phosphorylation.

An increasing number of mechanisms of resistance to anti-BRAF and/or anti-MEK compounds are being described (Lito et al., 2013; Shi et al., 2013; Tentori et al., 2013). Here, eIF4F complex formation is identified as a unique convergence nexus of multiple molecular lesions driving drug resistance. Our data strongly suggest that eIF4F is a critical therapeutic target strategically located downstream of major cell signaling cascades.

## REFERENCES

Blagden SP, Willis AE. The biological and therapeutic relevance of mRNA translation in cancer. Nat Rev Clin Oncol 2011; 8: 280-91.

Chapman PB, Hauschild A, Robert C, Haanen JB, Ascierto P, Larkin J, Dummer R, Garbe C, Testori A, Maio M, Hogg D, Lorigan P, Lebbe C, Jouary T, Schadendorf D, Ribas A, O'Day SJ, Sosman JA, Kirkwood JM, Eggermont AM, Dreno B, Nolop K, Li J, Nelson B, Hou J, Lee RJ, Flaherty KT, McArthur GA; BRIM-3 Study Group. Improved survival with vemurafenib in melanoma with BRAF V600E mutation. N Engl J Med. 2011 Jun 30;364(26):2507-16

Flaherty KT, Puzanov I, Kim KB, Ribas A, McArthur GA, Sosman JA, O'Dwyer PJ, Lee RJ, Grippo JF, Nolop K, Chapman PB. Inhibition of mutated, activated BRAF in metastatic melanoma. N Engl J Med. 2010 Aug 26;363(9):809-19.

Flaherty KT, Infante JR, Daud A, Gonzalez R, Kefford RF, Sosman J, Hamid O, Schuchter L, Cebon J, Ibrahim N, Kudchadkar R, Burris HA 3rd, Falchook G, Algazi A, Lewis K, Long GV, Puzanov I, Lebowitz P, Singh A, Little S, Sun P, Allred A, Ouellet D, Kim KB, Patel K, Weber J. Combined BRAF and MEK inhibition in melanoma with BRAF V600 mutations. N Engl J Med. 2012 Nov;367(18):1694-703.

Fedorenko IV, Paraiso KH, Smalley KS. Acquired and intrinsic BRAF inhibitor resistance in BRAF V600E mutant melanoma. Biochem Pharmacol. 2011 Aug 1;82(3):201-9

Hauschild A, Grob JJ, Demidov LV, Jouary T, Gutzmer R, Millward M, Rutkowski P, Blank CU, Miller WH Jr, Kaempgen E, Martin-Algarra S, Karaszewska B, Mauch C, Chiarion-Sileni V, Martin AM, Swann S, Haney P, Mirakhur B, Guckert ME, Goodman V, Chapman PB. Dabrafenib in BRAF-mutated metastatic melanoma: a multicentre, open-label, phase 3 randomised controlled trial. Lancet. 2012 Jul 28;380(9839):358-65

Hou J, Lam F, Proud C, Wang S. Targeting Mnks for cancer therapy. Oncotarget. 2012 Feb; 3(2): 118-131.

Lito, P., Rosen, N. & Solit, D. B. Tumor adaptation and resistance to RAF inhibitors. Nature medicine 19, 1401-1409, doi:10.1038/nm.3392 (2013).

Moerke NJ, Aktas H, Chen H, Cantel S, Reibarkh MY, Fahmy A, Gross JD, Degterev A, Yuan J, Chorev M, Halperin JA, Wagner G. Small-molecule inhibition of the interaction between the translation initiation factors eIF4E and eIF4G. Cell. 2007 Jan 26;128(2):257-67.

Montero-Conde, C. et al. Relief of feedback inhibition of HER3 transcription by RAF and MEK inhibitors attenuates their antitumor effects in BRAF-mutant thyroid carcinomas. Cancer discovery 3, 520-533, doi:10.1158/2159-8290.CD-12-0531 (2013).

Oikonomou, E., Koc, M., Sourkova, V., Andera, L. & Pintzas, A. Selective BRAFV600E inhibitor PLX4720, requires TRAIL assistance to overcome oncogenic PIK3CA resistance. PloS one 6, e21632, doi:10.1371/joumal.pone.0021632 (2011).

Prahallad, A. et al. Unresponsiveness of colon cancer to BRAF(V600E) inhibition through feedback activation of EGFR. Nature 483, 100-103, doi:10.1038/nature10868 (2012).

Straussman R, Morikawa T, Shee K, Barzily-Rokni M, Qian ZR, Du J, Davis A, Mongare MM, Gould J, Frederick DT, Cooper ZA, Chapman PB, Solit DB, Ribas A, Lo RS, Flaherty KT, Ogino S, Wargo JA, Golub TR. Tumour microenvironment elicits innate resistance to RAF inhibitors through HGF secretion. Nature. 2012 Jul 26;487(7408):500-4.

Tentori, L., Lacal, P. M. & Graziani, G. Challenging resistance mechanisms to therapies for metastatic melanoma. Trends in pharmacological sciences, doi:10.1016/j.tips.2013.10.003 (2013).

Wilson TR, Fridlyand J, Yan Y, Penuel E, Burton L, Chan E, Peng J, Lin E, Wang Y, Sosman J, Ribas A, Li J, Moffat J, Sutherlin DP, Koeppen H, Merchant M, Neve R, Settleman J. Widespread potential for growth-factor-driven resistance to anticancer kinase inhibitors. Nature. 2012 Jul 26;487(7408):505-9

Zindy P et al. "Formation of the eIF4F Translation-Initiation Complex Determines Sensitivity to Anticancer Drugs Targeting the EGFR and HER2 Receptors", Cancer Research 2011; 71(12): 4068-4073

## Claims

1. A method of evaluating sensitivity or resistance of a tumor to an antitumoral agent, comprising assessing the quantity of eiF4E-eiF4G complex (Cap-ON complex) and the quantity of eiF4E-4EBP complex (Cap-OFF complex) in said tumor or in tumor cells derived therefrom and correlating the relative amount of the Cap-OFF complex in comparison to the Cap-ON complex with sensitivity or resistance to the antitumoral agent.

2. The method according to claim 1, wherein the tumor is a melanoma, in particular a metastatic melanoma, a renal cancer, a colorectal cancer, a breast cancer or a lung cancer.

3. The method according to claim 2, wherein the tumor is a metastatic melanoma harboring a mutation in BRAF, in particular BRAF V600E mutation.

4. The method according to any one of claims 1 to 3, wherein the antitumoral agent is selected in the group consisting of BRAF inhibitors, MEK1/2 (or MAPK) inhibitors, mTOR or Akt inhibitors, VEGFR inhibitors and EGFR inhibitors.

5. The method according to any one of claims 1 to 4, wherein the antitumoral agent is an inhibitor of BRAF selected in the group consisting of sorafenib, PLX-4720 and LGX818.

6. The method according to any one of claims 1 to 4, wherein the antitumoral agent is an inhibitor of MEK selected in the group consisting of PD0325901, selumetinib and GDC0973.

7. The method according to any one of claims 1 to 6, wherein assessing the quantity of the Cap-ON and Cap-OFF complexes comprises using a proximity ligation assay (PLA) or a CAP-binding assay.

8. The method according to any one of claims 1 to 7, wherein the method further comprises a step of comparing the relative amount of the Cap-OFF and Cap-ON complexes in the tumor to a reference level.

9. A method of selecting an appropriate treatment of a cancer for a subject having a tumor, which method comprises a step of determining the amount of the Cap-OFF and Cap-ON forms of the eiF4F complex in a biological sample of said subject, a high Cap-OFF/Cap-ON ratio in the biological sample being the indication that a treatment with an antitumoral agent will be efficient in the subject, a low Cap-OFF/Cap-ON ratio in the biological sample being the indication that a treatment with an antitumoral agent will not be efficient in the subject.

10. A method for screening or identifying a compound suitable for improving the treatment of a cancer with an antitumoral agent in a subject having a tumor, said method comprising determining the ability of a test compound to increase the Cap-OFF/Cap-ON eiF4F complex ratio.

**Patentansprüche**

1. Ein Verfahren zur Evaluierung von Sensitivität oder Resistenz eines Tumors gegen ein Antitumormittel, umfassend Abschätzung der Menge an eiF4E-eiF4G-Komplex (Cap-ON-Komplex) und der Menge an eiF4E-4EBP-Komplex (Cap-OFF-Komplex) in besagtem Tumor oder in davon abstammenden Tumorzellen und Korrelation der relativen Menge des Cap-OFF-Komplexes im Vergleich zu dem Cap-ON-Komplex mit einer Sensitivität oder Resistenz gegen das Antitumormittel.

2. Das Verfahren gemäß Anspruch 1, wobei der Tumor ein Melanom, insbesondere ein metastasierendes Melanom, ein Nierenkrebs, ein Kolorektalkrebs, ein Brustkrebs oder ein Lungenkrebs ist.

3. Das Verfahren gemäß Anspruch 2, wobei der Tumor ein metastasierendes Melanom mit einer Mutation in BRAF, insbesondere BRAF V600E-Mutation ist.

4. Das Verfahren gemäß einem der Ansprüche 1 bis 3, wobei das Antitumormittel aus der Gruppe ausgewählt ist, bestehend aus BRAF-Hemmern, MEK1/2- (oder MAPK)-Hemmern, mTOR- oder Akt-Hemmern, VEGFR-Hemmern und EGFR-Hemmern.

5. Das Verfahren gemäß einem der Ansprüche 1 bis 4, wobei das Antitumormittel ein Hemmer von BRAF ist, ausgewählt aus der Gruppe, bestehend aus Sorafenib, PLX-4720 und LGX818.

6. Das Verfahren gemäß einem der Ansprüche 1 bis 4, wobei das Antitumormittel ein Hemmer von MEK ist, ausgewählt aus der Gruppe, bestehend aus PD0325901, Selumetinib und GDC0973.

7. Das Verfahren gemäß einem der Ansprüche 1 bis 6, wobei die Abschätzung der Menge der Cap-ON- und Cap-OFF-Komplexe eine Verwendung eines Proximity Ligation Assays (PLA) oder eines CAP-Bindungsassays umfasst.

8. Das Verfahren gemäß einem der Ansprüche 1 bis 7, wobei das Verfahren außerdem einen Schritt des Vergleichs der relativen Menge der Cap-OFF- und Cap-ON-Komplexe in dem Tumor mit einem Referenzwert umfasst.

**9.** Ein Verfahren zur Auswahl einer geeigneten Behandlung eines Krebses bei einer Person mit einem Tumor, wobei das Verfahren einen Schritt der Bestimmung der Menge der Cap-OFF- und Cap-ON-Formen des eiF4F-Komplexes in einer biologischen Probe besagter Person umfasst, wobei ein hohes Cap-OFF/Cap-ON-Verhältnis in der biologischen Probe anzeigt, dass eine Behandlung mit einem Antitumormittel in der Person wirksam ist, ein niedriges Cap-OFF/Cap-ON-Verhältnis in der biologischen Probe anzeigt, dass eine Behandlung mit einem Tumormittel in der Person nicht wirksam ist.

**10.** Ein Verfahren zum Screening oder zur Identifizierung einer Verbindung, die zur Verbesserung der Behandlung eines Krebses mit einem Antitumormittel in einer Person mit einem Krebs geeignet ist, wobei besagtes Verfahren eine Bestimmung der Fähigkeit einer Testverbindung umfasst, das Cap-OFF/Cap-ON-eiF4F-Komplex-Verhältnis zu erhöhen.

**Revendications**

**1.** Méthode d'évaluation de la sensibilité ou de la résistance d'une tumeur à un agent antitumoral, comprenant la détermination de la quantité de complexe eiF4E-eiF4G (complexe Cap-ON) et de la quantité de complexe eiF4E-4EBP (complexe Cap-OFF) dans ladite tumeur ou dans des cellules tumorales en dérivant, et la corrélation de la quantité relative du complexe Cap-OFF comparé au complexe Cap-ON avec la sensibilité ou la résistance à l'agent antitumoral.

**2.** Méthode selon la revendication 1, dans laquelle la tumeur est un mélanome, en particulier un mélanome métastasique, un cancer rénal, un cancer colorectal, un cancer du sein ou un cancer du poumon.

**3.** Méthode selon la revendication 2, dans laquelle la tumeur est un mélanome métastasique hébergeant une mutation dans BRAF, en particulier la mutation V600E de BRAF

**4.** Méthode selon l'une quelconque des revendications 1 à 3, dans laquelle l'agent antitumoral est choisi dans le groupe constitué par les inhibiteurs de BRAF, les inhibiteurs de MEK1/2 (ou MAPK), les inhibiteurs de mTOR ou d'Akt, les inhibiteurs de VEGFR et les inhibiteurs d'EGFR.

**5.** Méthode selon l'une quelconque des revendications 1 à 4, dans laquelle l'agent antitumoral est un inhibiteur de BRAF choisi dans le groupe constitué par le sorafénib, PLX-4720 et LGX818.

**6.** Méthode selon l'une quelconque des revendications 1 à 4, dans laquelle l'agent antitumoral est un inhibiteur de MEK choisi dans le groupe constitué par PD0325901, le sélumétinib et GDC0973.

**7.** Méthode selon l'une quelconque des revendications 1 à 6, dans laquelle la détermination de la quantité des complexes Cap-ON et Cap-OFF comprend l'utilisation d'un test de ligature de proximité (PLA) ou d'un test de liaison à CAP.

**8.** Méthode selon l'une quelconque des revendications 1 à 7, laquelle méthode comprend en outre une étape de comparaison de la quantité relative des complexes Cap-OFF et Cap-ON dans la tumeur avec un niveau de référence.

**9.** Méthode de sélection d'un traitement approprié d'un cancer chez un sujet ayant une tumeur, laquelle méthode comprend une étape de détermination de la quantité des formes Cap-OFF et Cap-ON du complexe d'eiF4F dans un échantillon biologique dudit sujet, un rapport Cap-OFF/Cap-ON élevé dans l'échantillon biologique étant une indication qu'un traitement avec un agent antitumoral sera efficace chez le sujet, un rapport Cap-OFF/Cap-ON faible dans l'échantillon biologique étant une indication qu'un traitement avec un agent antitumoral ne sera pas efficace chez le sujet.

**10.** Méthode de criblage ou d'identification d'un composé convenant pour améliorer le traitement d'un cancer avec un agent antitumoral chez un sujet ayant une tumeur, ladite méthode comprenant la détermination de la capacité d'un composé de test à augmenter le rapport Cap-OFF/Cap-ON de complexe d'eiF4F.

**FIGURE 1**

**FIGURE 2**

FIGURE 3

**FIGURE 4**

**FIGURE 5**

FIGURE 6

Figure 7

Figure 8

Figure 8 (cont.)

**Figure 9**

**Figure 9 (cont.)**

Figure 10

Figure 11

**Figure 11 (cont.)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 13016658 A
- WO 12066002 A
- WO 10060891 A

### Non-patent literature cited in the description

- **BLAGDEN SP ; WILLIS AE.** The biological and therapeutic relevance of mRNA translation in cancer. *Nat Rev Clin Oncol,* 2011, vol. 8, 280-91
- **CHAPMAN PB ; HAUSCHILD A ; ROBERT C ; HAANEN JB ; ASCIERTO P ; LARKIN J ; DUMMER R ; GARBE C ; TESTORI A ; MAIO M.** BRIM-3 Study Group. Improved survival with vemurafenib in melanoma with BRAF V600E mutation. *N Engl J Med.,* 30 June 2011, vol. 364 (26), 2507-16
- **FLAHERTY KT ; PUZANOV I ; KIM KB ; RIBAS A ; MCARTHUR GA ; SOSMAN JA ; O'DWYER PJ ; LEE RJ ; GRIPPO JF ; NOLOP K.** Inhibition of mutated, activated BRAF in metastatic melanoma. *N Engl J Med.,* 26 August 2010, vol. 363 (9), 809-19
- **FLAHERTY KT ; INFANTE JR ; DAUD A ; GONZALEZ R ; KEFFORD RF ; SOSMAN J ; HAMID O ; SCHUCHTER L ; CEBON J ; IBRAHIM N.** Combined BRAF and MEK inhibition in melanoma with BRAF V600 mutations. *N Engl J Med.,* November 2012, vol. 367 (18), 1694-703
- **FEDORENKO IV ; PARAISO KH ; SMALLEY KS.** Acquired and intrinsic BRAF inhibitor resistance in BRAF V600E mutant melanoma. *Biochem Pharmacol.,* 01 August 2011, vol. 82 (3), 201-9
- **HAUSCHILD A ; GROB JJ ; DEMIDOV LV ; JOUARY T ; GUTZMER R ; MILLWARD M ; RUTKOWSKI P ; BLANK CU ; MILLER WH JR ; KAEMPGEN E.** Dabrafenib in BRAF-mutated metastatic melanoma: a multicentre, open-label, phase 3 randomised controlled trial. *Lancet,* 28 July 2012, vol. 380 (9839), 358-65
- **HOU J ; LAM F ; PROUD C ; WANG S.** Targeting Mnks for cancer therapy. *Oncotarget,* February 2012, vol. 3 (2), 118-131
- **LITO, P. ; ROSEN, N. ; SOLIT, D. B.** Tumor adaptation and resistance to RAF inhibitors. *Nature medicine,* 2013, vol. 19, 1401-1409
- **MOERKE NJ ; AKTAS H ; CHEN H ; CANTEL S ; REIBARKH MY ; FAHMY A ; GROSS JD ; DEGTEREV A ; YUAN J ; CHOREV M.** Small-molecule inhibition of the interaction between the translation initiation factors eIF4E and eIF4G. *Cell,* 26 January 2007, vol. 128 (2), 257-67
- **MONTERO-CONDE, C. et al.** Relief of feedback inhibition of HER3 transcription by RAF and MEK inhibitors attenuates their antitumor effects in BRAF-mutant thyroid carcinomas. *Cancer discovery,* 2013, vol. 3, 520-533
- **OIKONOMOU, E. ; KOC, M. ; SOURKOVA, V. ; ANDERA, L. ; PINTZAS, A.** Selective BRAFV600E inhibitor PLX4720, requires TRAIL assistance to overcome oncogenic PIK3CA resistance. *PloS one,* 2011, vol. 6, e21632
- **PRAHALLAD, A. et al.** Unresponsiveness of colon cancer to BRAF(V600E) inhibition through feedback activation of EGFR. *Nature,* 2012, vol. 483, 100-103
- **STRAUSSMAN R ; MORIKAWA T ; SHEE K ; BARZILY-ROKNI M ; QIAN ZR ; DU J ; DAVIS A ; MONGARE MM ; GOULD J ; FREDERICK DT.** Tumour micro-environment elicits innate resistance to RAF inhibitors through HGF secretion. *Nature,* 26 July 2012, vol. 487 (7408), 500-4
- **TENTORI, L. ; LACAL, P. M. ; GRAZIANI, G.** Challenging resistance mechanisms to therapies for metastatic melanoma. *Trends in pharmacological sciences,* 2013
- **WILSON TR ; FRIDLYAND J ; YAN Y ; PENUEL E ; BURTON L ; CHAN E ; PENG J ; LIN E ; WANG Y ; SOSMAN J.** Widespread potential for growth-factor-driven resistance to anticancer kinase inhibitors. *Nature,* 26 July 2012, vol. 487 (7408), 505-9
- **ZINDY P et al.** Formation of the eIF4F Translation-Initiation Complex Determines Sensitivity to Anticancer Drugs Targeting the EGFR and HER2 Receptors. *Cancer Research,* 2011, vol. 71 (12), 4068-4073